# EUROPEAN PATENT APPLICATION

(11) **EP 2 305 667 A2**
(43) Date of publication of application: **06.04.2011**
(21) Application number: 11151589.6
(22) Date of filing: 17.07.2009
(51) Int. Cl.: C07D 401/14

(54) **Nilotinib intermediates and preparation thereof**

(30) Priority: 17.07.2008 US 81464 P; 24.07.2008 US 83424 P; 20.08.2008 US 90368 P; 24.11.2008 US 117478 P; 19.03.2009 US 161670 P; 13.05.2009 US 168822 P; 22.05.2009 US 171706 P
(62) Divisional of application: 09790590.5
(71) Applicant: Teva Pharmaceutical Industries Ltd., 49131 Petah Tiqva (IL)
(72) Inventor: Wang, Yanling, Pudong Shanhai (CN); Li, Jie, Pudong Shanghai (CN); Kansal, Vinod, Kumar, 121003, Faridabad Haryana (IN); Zhu, Jirang, Pudong Shanghai (CN); Lifshitz-Liron, Revital, Herzlia (IL); Mistry, Dhirenkumar, N., 393145, Rajpipla Gujarat (IN); Vasoya, Sanjay, L., 360002, Dist-rajkot Gujarat (IN); Ariyamuthu, Sundaraselvan, 627006, Tirunelveli Tamilnada (IN); Pilarski, Gideon, 58417 Holon (IL); He, Xungui, 201204 Pudong Shanghai (CN)
(74) Representative: Wong, Kit Yee

(57) **Abstract**

Nilotinib•3HCl and its crystalline forms are described, and processes for the preparation of the same.

## Description

### CROSS-REFERENCE TO RELATED APPLICATIONS

This application claims the benefit of U.S. Provisional Patent Application Serial. Nos. 61/081,464, filed July 17, 2008; 61/083,424, filed July 24, 2008; 61/090,368, filed August 20, 2008; 61/117,478, filed November 24, 2008; 61/161,670, filed March 19, 2009; 61/171,706, filed April 22, 2009; 61/168,822, filed April 13, 2009, which are incorporated herein by reference.

### FIELD OF INVENTION

The present invention is directed to preparation of Nilotinib by a one-pot process, intermediates of Nilotinib, Nilotinib·3HC1 and its crystalline forms.

### BACKGROUND OF THE INVENTION

Nilotinib, 4-methyl-N-[3-(4-methyl-1H-imidazol-1-yl)-5-(trifluoromethyl)phenyl]-3-[[4-(3-pyridinyl)-2-pyrimidinyl]amino]-benzamide, having the following formula 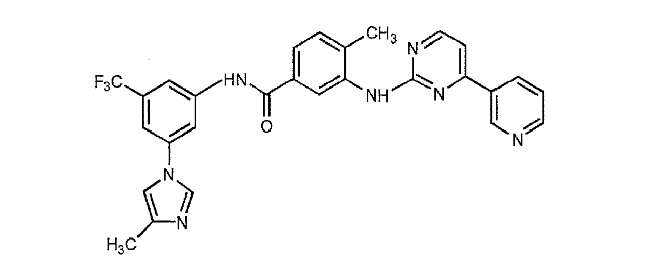 is a tyrosine kinase inhibitor used for the treatment of drug-resistant chronic myelogenous leukemia (CML), and in particular, for the treatment of chronic phase and accelerated phase Philadelphia chromosome positive chronic myeloid leukemia (CML) in adult patients whose disease has progressed on or who cannot tolerate other therapies that included imatinib. Nilotinib is administered as a hydrochloride salt in forms of capsules that are marketed in the USA and the EU under the name Tasigna®.

US patent no. 7,169,791 ("US '791 ") and its parallel PCT publication WO 2004/005281, the journal article in Synthesis, 2007, vol 14, pp 2121-2124, as well as PCT publication nos.: WO 2006/135640, WO 2006/135641 ("WO '641 "), WO 2007/018325 and WO 2007/017734, report processes for preparing Nilotinib intermediate, 3-(trifluoromethyl)-5-(4-methyl-1H-imidazole-1-yl)-benzeneamine of formula I 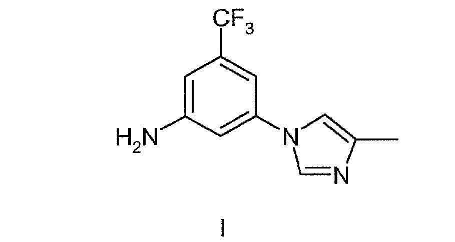 by reacting 3-bromo-5-trifluoromethylaniline of formula II and 4-methylimidazole of formula III in the presence of a non-alkaline hydroxide inorganic base, such as potassium carbonate, cesium carbonate and sodium hydride, a copper (I) salt, such as copper iodide and a complexing amine ligand, such as ethylene diamine. The process can be illustrated by the following scheme:

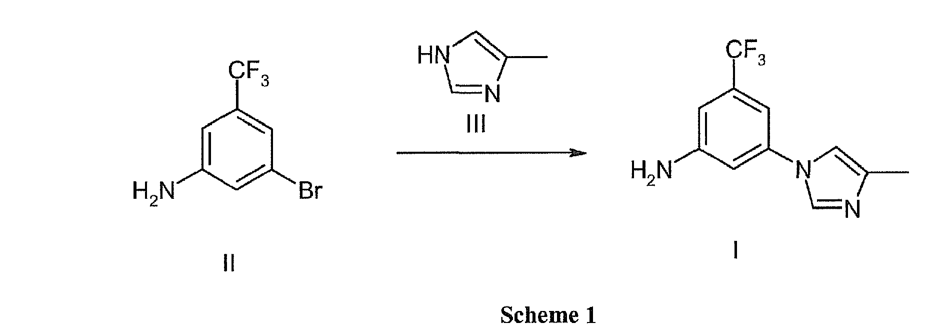

The journal article in Synthesis, 2007, Vol 14, pp 2121-2124, describes a purification process of 3-(trifluoromethyl-5-(4-methyl-1H-imidazole-1-yl)-beuzeneamine of formula I.

US '791 describes processes for preparing Nilotinib and its different intermediates, using di-ethyl cyano phosphate, as described in the following scheme:

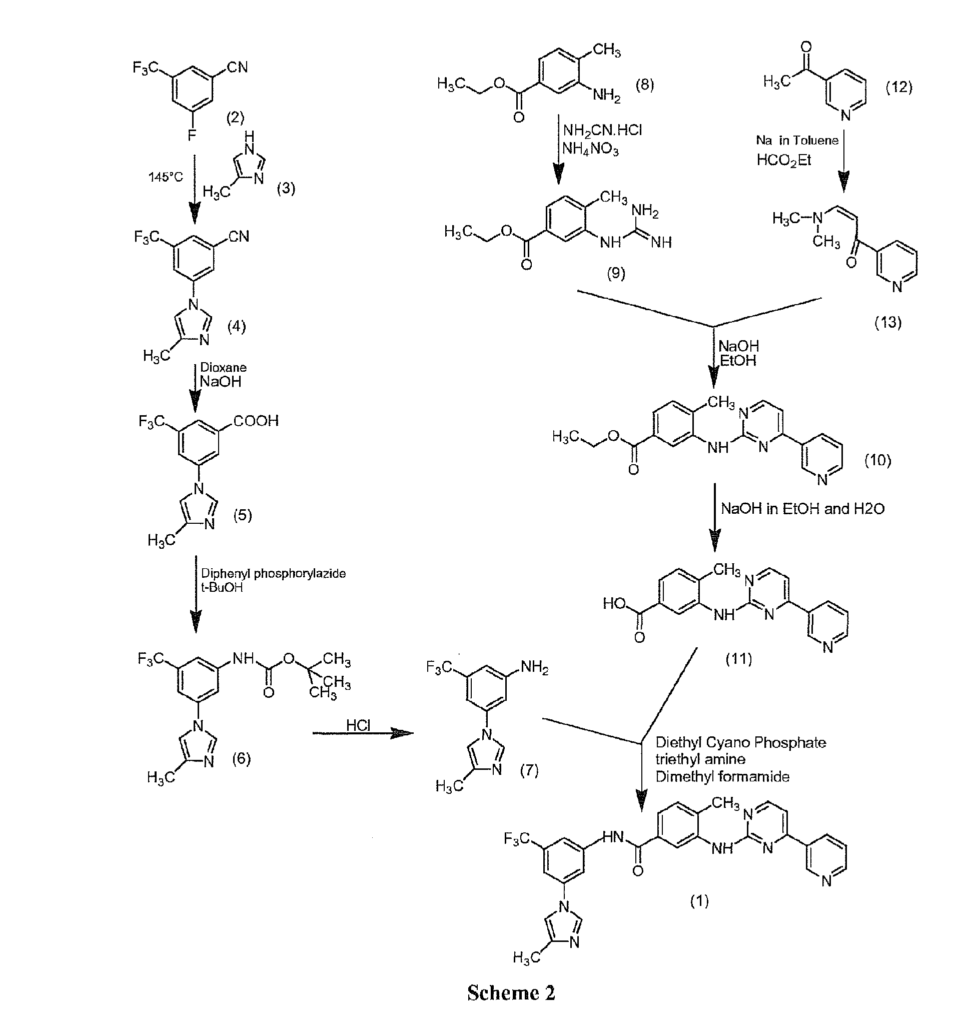

WO '641 further describes a process for preparing Nilotinib according to the following scheme:

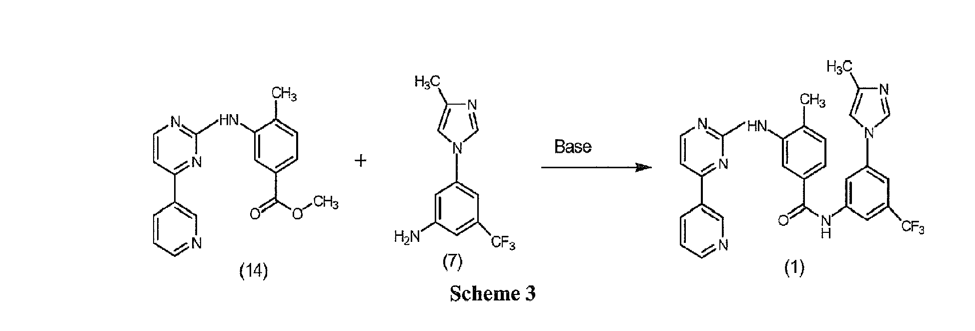

The present invention provides improved processes to prepare and/or purify 3-(trifluoromethyl)-5-(4-methyl-1H-imidazole-1-yl)-benzeneamine of formula I without requiring the use of column chromatography, and thus can be easily applied to large scale manufacture, as well as new intermediates of Nilotinib, which result in higher yields in the preparation of Nilotinib.

PCT publications WO 2007/015870 ("WO'870") and WO 2007/015871 1 ("WO'871") describe several Nilotinib salts including crystalline forms of nilotinib free base, Nilotinib hydrochloride and Nilotinib Sulfate.

The present invention also relates to the solid state physical properties of Nilotinib•3HCl, 4-methyl-N-[3-(4-methyl-1H-imidazol-1-yl)-5-(trifluoromethyl)phenyl]-3-[[4-(3-pyridinyl)-2-pyrimidinyl]amino]-benzamide trihydrochloride. These properties can be influenced by controlling the conditions under which Nilotinib-3HC1 is obtained in solid form. Solid state physical properties include, for example, the flowability of the milled solid. Flowability affects the ease with which the material is handled during processing into a pharmaceutical product. When particles of the powdered compound do not flow past each other easily, a formulation specialist must necessitate the use of glidants such as colloidal silicon dioxide, talc, starch, or tribasic calcium phosphate.

Another important solid state property of a pharmaceutical compound is its rate of dissolution in aqueous fluid. The rate of dissolution of an active ingredient in a patient's stomach fluid can have therapeutic consequences since it imposes an upper limit on the rate at which an orally administered active ingredient can reach the patient's bloodstream. The rate of dissolution is also a consideration in formulation syrups, elixirs, and other liquid medicaments. The solid state form of a compound can also affect its behavior on compaction and its storage stability.

These practical physical characteristics are influenced by the conformation and orientation of molecules in the unit cell, which define a particular polymorphic form of a substance. The polymorphic form can give rise to thermal behavior different from that of the amorphous material or another polymorphic form. Thermal behavior is measured in the laboratory by such techniques as capillary melting point, thermogravimetric analysis ("TGA"), and differential scanning calorimetry ("DSC") and can be used to distinguish some polymorphic forms from others. A particular polymorphic form can also give rise to distinct spectroscopic properties that can be detectable by powder x-ray crystallography, solid state ^{l3}C NMR spectroscopy, and infrared spectrometry.

Generally, a crystalline solid has improved chemical and physical stability over the amorphous form, and forms with low crystallinity. Crystalline forms may also exhibit improved solubility, hygroscopicity, bulk properties, and/or flowability.

The discovery of new polymorphic forms of a pharmaceutically useful compound provides a new opportunity to improve the performance characteristics of a pharmaceutical product. It enlarges the repertoire of materials that a formulation scientist has available for designing, for example, a pharmaceutical dosage form of a drug with a targeted release profile or other desired characteristic.

There is a need in the art for new intermediates of Nilotinib and processes for their preparation, new processes for preparing Nilotinib and new crystalline forms of Nilotinib•3HCl salt and processes for the preparation thereof.

### SUMMARY OF THE INVENTION

In one embodiment, the present invention provides a process for preparing 3-(trifluoromethyl)-5-(4-methyl-1H-imidazole-1-yl)-benzeneamine of formula I, 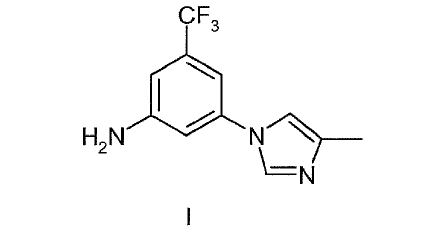 comprising reacting 3-bromo-5- trifluoromethylaniline of formula II, 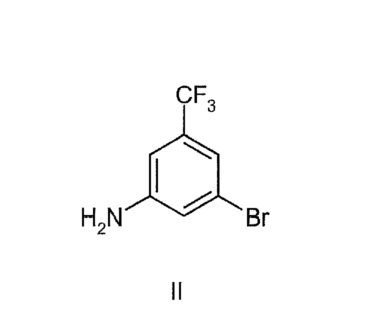 4-methylimidazole of formula III, 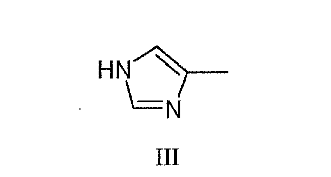 a base selected from a group consisting of: an alkaline metal hydroxide, an alkaline earth metal hydroxide and ammonium hydroxide; and a water absorbing agent.

In another embodiment, the present invention provides a process for crystallizing the compound of formula I from a mixture of ethyl acetate and petroleum ether comprising dissolving 3-(trifluoromethyl-5-(4-methyl-1H-imidazole-1-yl)-benzeneamine of formula I in ethyl acetate; adding petroleum ether to obtain a suspension and isolating.

In yet another embodiment, the present invention further provides a process for purifying the intermediate of formula I by recrystallizing it from a mixture of IPA and water or a mixture of ethanol and water.

In one embodiment, the present invention provides a process for preparing Nilotinib and salt thereof of the following formula 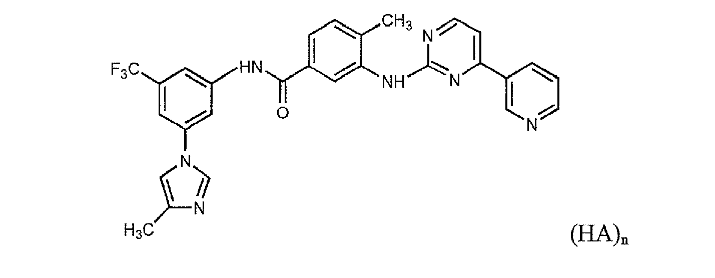 comprising: preparing 3-(trifluoromethyl)-5-(4-methyl-1H-imidazole-1-yl)-benzeneamine of formula I according to the processes of the present and converting it to Nilotinib or a salt thereof
wherein, n is either 0 or 1, and HA is an acid, preferably, HCI.

In another embodiment, the present invention provides N-(3-Bromo-5-trifluoromethylphenyl)-4-methyl-3-[[4-(3-pyridinyl)-2-pyrimidinyl]amino]benzamide, a Nilotinib intermediate of formula IV, having the following structure:

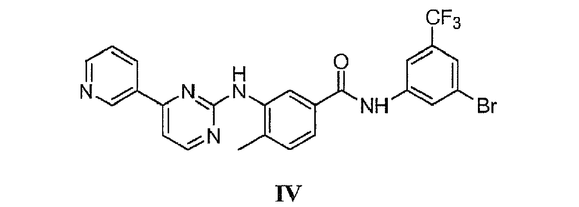

In another embodiment, the present invention provides an isolated N-(3-Bromo-5-trifluoromethylphenyl)-4-methyl-3-[[4-(3-pyridinyl)-2-pyrimidinyl]amino]benzamide, a Nilotinib intermediate of formula IV.

In yet another embodiment, the present invention provides processes for preparing the compound of formula IV. One process comprises: combining a compound of formula X 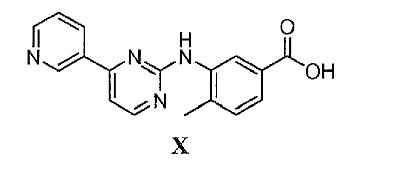 with a solvent selected from the group consisting of: aromatic hydrocarbon, a C₄-C₆ cyclic or aliphatic ether, a chlorinated solvent selected from the group consisting of: dichloromethane, dichloroethane, chlorobenzene and chloroform, DMA, DMF, DMSO and n-methyl-pyrrolidone, a compound selected from the group consisting of: thionyl chloride ("SOC1₂"), thionyl bromide, oxalyl chloride, oxalyl bromide, phosphorous trichloride, phosphorous tribromide, phosphorous pentachloride, phosphorous pentabromide, C₁-C₅ carboxylic acid and C₂-C₈ anhydride and activated reagent such as Di-tert-Butyl dicarbonate ("BOC"); a compound of formula II, and a base selected from the group consisting of: organic C₂-C₅ secondary and tertiary amines, inorganic bases and mixtures thereof.

In one embodiment, the present invention provides another process for preparing the Nilotinib intermediate of formula IV comprising: combining a compound of formula X with a compound of formula II, a coupling reagent preferably selected from the group consisting of: diethylcyanophosphonate, 1-hydroxybenzotriazole/ 1-[3-(Dimethylamino)propyl]-3-ethylcarbodiimide HC1 ("HOBt/EDCI"), 1,1'-carbonyldiimidazole ("CDI"), N, N'-Dicyclohexylcarbodiimide ("DCC") and Bis(2-oxo-3-oxazolidinyl)phosphonic chloride ("BOP-C"1); and an aprotic polar solvent preferably, selected from the group consisting of: DMF, DMSO and acetonitrile.

In another embodiment, the present invention provides processes for preparing Nilotinib or salt thereof from a Nilotinib intermediate of formula IV. The conversion may be done by combining a compound of formula IV, 4-methylimidazole of formula III, a base, which is preferably selected from a non-hydroxylic base, and more preferably wherein the base is selected from the group consisting of: DMAP, 2,3,4,6,7,8,9,10-Octahydropyrimidol[1,2-a]azepine ("DBU"), K₂C0₃, C_{S2}CO₃, Na₂C0₃, KHC0₃, and NaHC0₃ , and a solvent selected from the group consisting of: DMSO and DMF.

In yet another embodiment, the present invention provides a purification process comprising combining Nilotinib with DMF or methylene chloride ("DCM") to obtain a first reaction mixture; filtering; washing; adding methanol/water to obtain a second reaction mixture; filtering; washing and drying.

In one embodiment, the present invention provides another purification process comprising combining nilotinib with dichloromethane/methanol to obtain a reaction mixture; filtering; and drying it.

In another embodiment, the present invention provides a one-pot process for preparing Nilotinib, 4-methyl-N-[3-(4-methyl-1H-imidazol-1-yl)-5-(trifluoromethyl)phenyl]-3-[[4-(3-pyridinyl)-2-pyrimidinyl]amino]-benzamide, comprising: combining 3-(trifluoromethyl)-5-(4-methyl-1H-imidazole-1-yl)-benzeneamine of formula I: 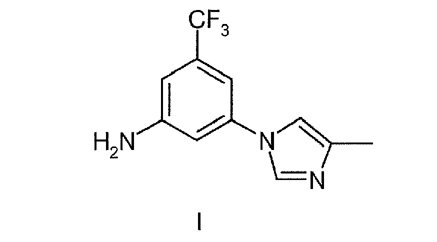 with 4-methyl-3-{[4-(pyridin-3-yl)pyrimidin-2-yl]amino}benzoic acid of formula X, having the following structure: 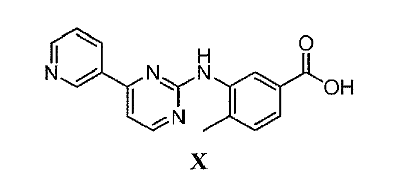 a solvent selected from the group consisting of: N-methyl pyrrolidone ("NMP"), dimethyl formamide ("DMF"), dimethylacetamide ("DMA"), and a chlorinated solvent such as CH₂C1₂, and a compound selected from the group consisting of: thionyl chloride ("SOCl₂"), thionyl bromide, oxalyl chloride, oxalyl bromide, phosphorous tri-chloride, phosphorous tri-bromide, phosphorous penta-chloride, phosphorous penta-bromide, C₁-C₅ carboxylic acid and C₂-C₈ anhydride and activated reagent such as Di-tert-Butyl dicarbonate ("BOC"); and adding a base selected from the group consisting of: NaOH, KOH, LiOH, K₂C0₃, Na₂C0₃, NaHC0₃, secondary amine, tertiary amine, NaH and CS₂CO₃ to obtain Nilotinib.

In yet another embodiment, the present invention provides a process for preparing Nilotinib·HCl comprising preparing Nilotinib according to the process of the present invention and converting it to Nilotinib·HCl. The conversion may be done, for example, by the process disclosed in US patent no. 7,169,791, which is incorporated herein by reference.

In one embodiment, the present invention provides a process for preparing Nilotinib intermediate of formula (X-M)•2HM, wherein M is an halogen, comprising: combining a compound of formula X with a halogenating agent selected from the group consisting of: thionyl chloride ("SOC1₂"), phosphorous oxychloride, phosphorous penta chloride, phosphorous trichloride, sulphuryl chloride, oxalyl chloride, N- bromosuccinimide and bromine.

In another embodiment, the present invention provides a process for preparing Nilotinib or a salt thereof by a process comprising: preparing the compound of formula (X-M)-2HM, wherein M is an halogen; and converting it to nilotinib.

In yet another embodiment, the present invention provides 3-(4-(pyridin-3-yl)pyrimidin-2-ylamino)-N-(3-(trifluoromethyl)-5-(4-methyl-1H-imidazol-1-yl)phenyl)-4-methyl benzamide tri hydrochloride, Nilotinib•3HC1, having the following structure:

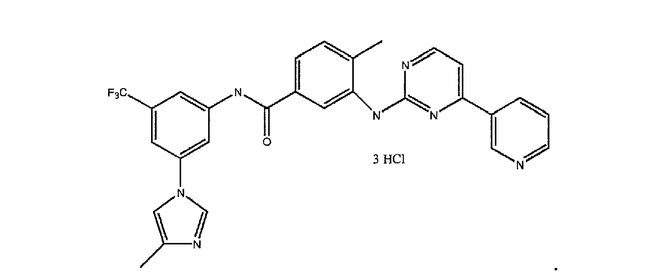

In one embodiment, the invention encompasses a solid form of Nilotinib•3HC1.

In another embodiment, the invention encompasses a crystalline form of Nilotinib•3HC1.

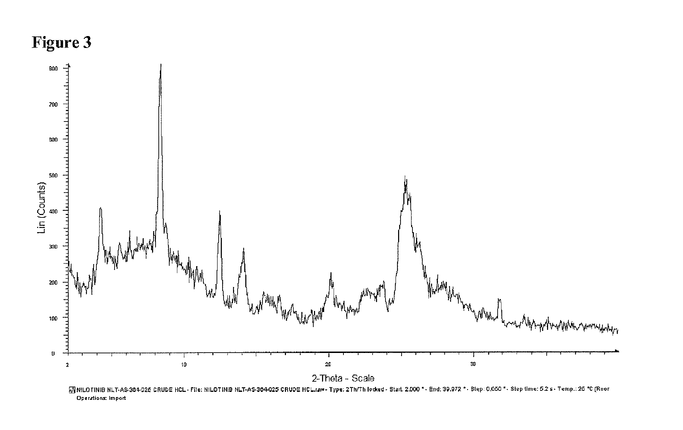

In yet another embodiment, the invention encompasses a crystalline form of Nilotinib•3HC1 characterized by data selected from the group consisting of: an x-ray powder diffraction pattern having peaks at about 8.3 and 25.2 degrees two theta ± 0.2 degrees two theta, an x-ray powder diffraction pattern substantially as depicted in Figure 3, and a combination thereof.

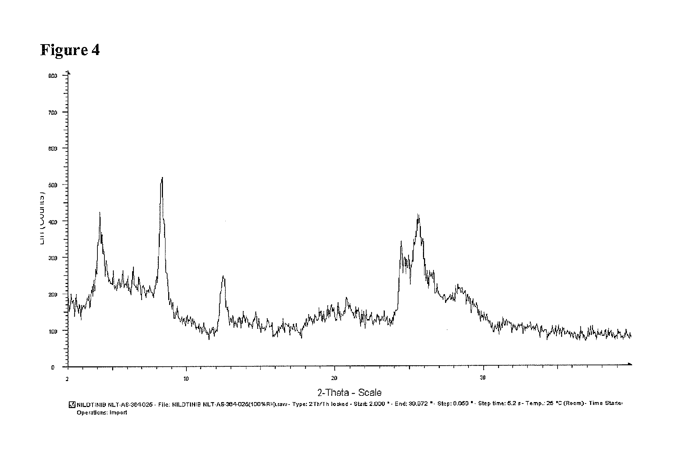

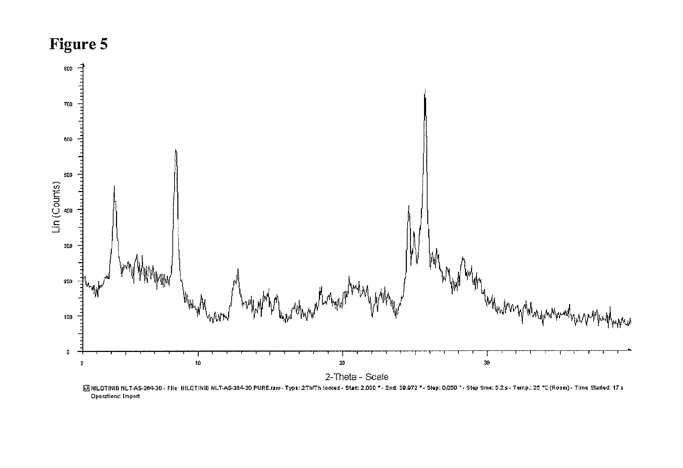

In one embodiment, the invention encompasses a crystalline form of Nilotinib•3HC1 characterized by data selected from the group consisting of: an x-ray powder diffraction pattern having peaks at about 4.1, 8.3 and 25.7 degrees two theta ± 0.2 degrees two theta, an x-ray powder diffraction pattern substantially as depicted in Figures 4 or 5, and a combination thereof.

In another embodiment, the invention encompasses a process for preparing Nilotinib•3HC1 comprising: combining the compound of formula (X-M)•2HM, wherein M is an halogen, with the compound of formula I, preferably in the presence of a solvent, wherein the solvent is preferably selected from the group consisting of: toluene, chloroform, dichloromethane, dimethylacetamide, THF, DMF, formamide, ethyl acetate, propyl acetate, butyl acetate, diethyl ether, methyl tert-butyl ether, hexane, cyclohexane, pentane, cyclopentane and mixtures thereof.

In yet another embodiment, the present invention provides another process for preparing Nilotinib•3HC1 comprising: adding hydrochloric acid source selected from the group consisting of: acetyl chloride, alcoholic hydrochloric acid, aqueous hydrochloric acid and hydrogen chloride gas to a first solvent selected from the group consisting of: C₁-C₄ alcohol, ketone, ester, nitrile and mixtures thereof; adding nilotinib free base; and adding a second solvent selected from the group consisting of: C₁-C₄ alcohol, ketone, ester, nitrile and mixtures thereof.

In one embodiment, the present invention provides a process for purifying Nilotinib·3HCl comprising: combining Nilotinib•3HC1 with water; filtering to obtain a filtrate; and crystallizing.

In another embodiment, the present invention provides a process for preparing Nilotinib or a salt thereof comprising: combining Nilotinib•3HC1 with water; and adding a base selected from the group consisting of: alkali metal hydroxide, alkali metal hydride, alkali metal carbonate, alkali metal bicarbonate, alkoxide, C₆-C₁₀ trialkyl amines such as triethyl amine and diisopropyl ethyl amine, and pyridine with a solvent selected from a group consisting of: methanol, ethanol, propanol, butanol, water and mixtures thereof, to obtain a precipitate.

In yet another embodiment, the present invention provides a purification process of Nilotinib comprising combining it with C₁-C₄ alcohol and inorganic base selected from the group consisting of: alkali metal hydroxide, alkali metal hydride, alkali metal carbonate, alkali metal bicarbonate and alkali metal alkoxide; heating; cooling; and recovering.

### BRIEF DESCRIPTION OF THE DRAWINGS

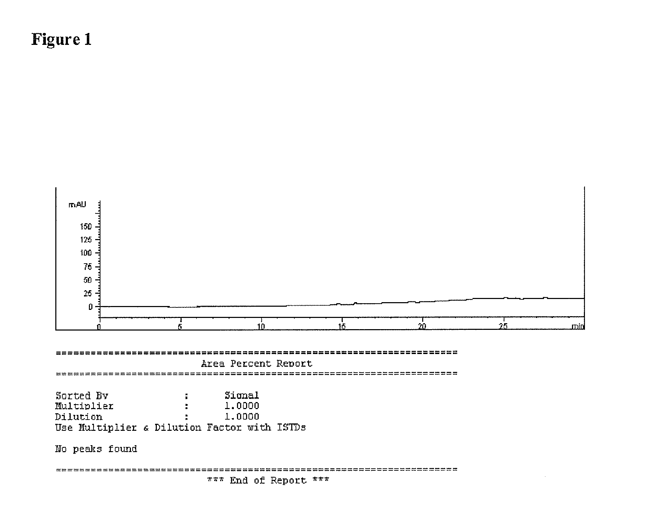

Figure 1: HPLC pattern of the blank (diluent only).

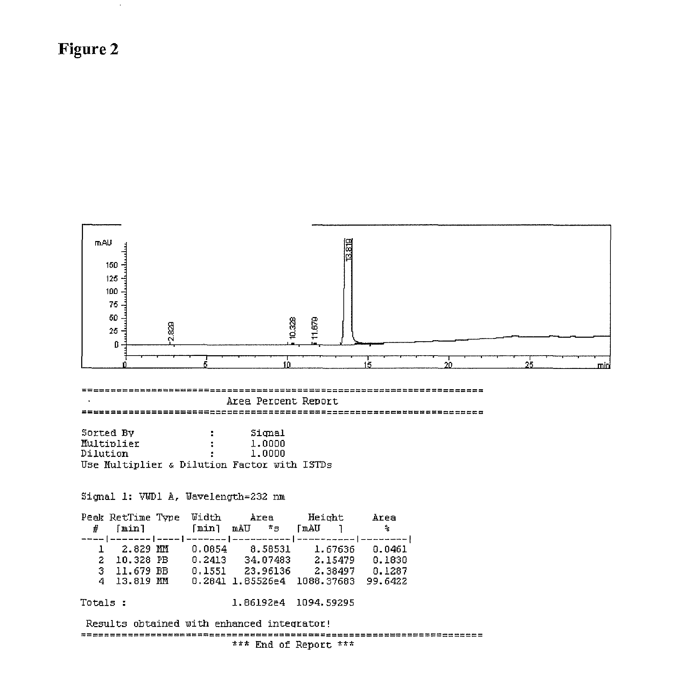

Figure 2: HPLC pattern of a sample of formula I obtained after recrystallization from IPA/water.

Figure 3: A characteristic x-ray powder diffractogram of Nilotinib•3HC1 crude.

Figure 4: A characteristic x-ray powder diffractogram of Nilotinib•3HC1 crude having water content at about 14.4% by weight.

Figure 5: A characteristic x-ray powder diffractogram of Nilotinib•3HC1 pure.

### DETAILED DESCRIPTION OF THE INVENTION

As used herein, the term "room temperature" refers to a temperature of about 15°C to about 30°C, more preferably, to a temperature of about 20°C to about 25°C.

As used herein, the term "overnight" refers to about 16 hours to about 24 hours.

As used herein, the term "water absorbing agent" refers to a material that reacts with free water to form hydroxide salt or hydrate.

As used herein, the term "reduced pressure" refers to a pressure of about 10mbar to about 50mbar.

As used herein, the term "isolated" in reference to the compound of formula IV corresponds to compound of formula IV that is physically separated from the reaction mixture, where it is formed.

In the processes for preparing 3-(trifluoromethyl)-5-(4-methyl-1H-imidazole-1-yl)-benzeneamine of formula I, of the following structure: 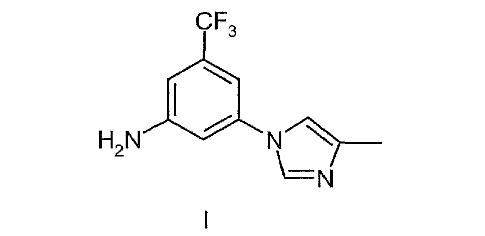 described in the prior art, the bases which are used either provide a non-homogeneous reaction mixture or are used in a large amount in order to ensure a complete reaction. The bases used in the prior art, such as cesium carbonate, are expensive and thus using them in large amounts result in a non economic process.

The present invention provides an improved process to prepare 3-(trifluoromethyl)-5-(4-methyl-1H-imidazole-1-yl)-benzeneamine of formula I, where a water absorbing agent is used. The use of water absorbing agent facilitates the promotion of the reaction towards completion by absorbing the water that is generated in the reaction, thus increases the conversion to 3-(trifluoromethyl)-5-(4-methyl-1H-imidazole-1-yl)-benzeneamine of formula I.

In addition, removing water from the reaction mixture helps avoid decomposition of the product, which occurs under the reaction conditions if water is present.

The process can be illustrated by the following scheme:

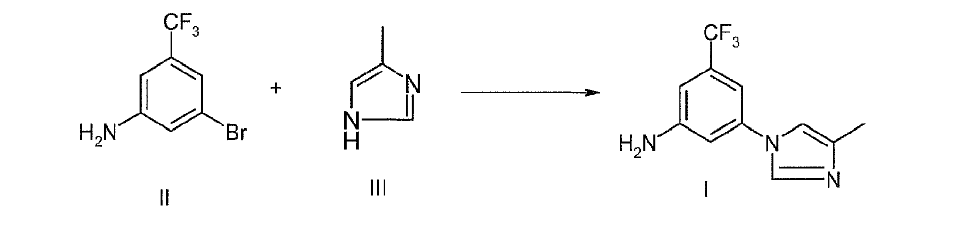

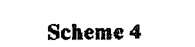

The process comprises combining 3-bromo-5- trifluoromethylaniline of formula II, 4-methylimidazole of formula **III,** a base selected from a group consisting of: an alkaline metal hydroxide, an alkaline earth metal hydroxide and ammonium hydroxide, and a. water absorbing agent.

Preferably, the alkaline metal hydroxide is NaOH, KOH or LiOH, more preferably, NaOH.

The use of alkaline metal hydroxide or ammonium hydroxide, which is soluble in the reaction mixture and therefore more available in reaction mixture, compared to K₂CO₃, used in the prior art, accelerates the reaction in comparison to that of the prior art.

Preferably, the water absorbing agent is selected from the group consisting of: CaO, BaO, MgO, Al₂O₃, CaO-SiO₂, CuS0₄, MgSO₄ and Na₂SO₄, more preferably, the water absorbent agent is CaO. Preferably, the water absorbing agent is an anhydrous salt.

Preferably, the reaction is done in the presence of a polar aprotic organic solvent.

Preferably, the solvent is dimethylsulfoxide ("DMSO"), dimethylformamide ("DMF") or dimethylacetamide ("DMA"), more preferably, DMSO.

Preferably, the reaction is done in the presence of an iodine salt selected from the group consisting of CuI, NaI and KI, more preferably, the iodine salt is CuI.

When the process is carried out in the presence of copper (I) iodide, optionally, the reaction is done in the presence of a copper complexing ligand, such as 8-hydroxyquinoline cyclohexanediamine or N,N-dimethyl-ethylenediamine.

Preferably, the process comprises precipitating the compound of formula I from a reaction mixture comprising the compounds of formula II and III, a polar aprotic organic solvent, an alkaline metal hydroxide, a water absorbing agent, CuI, and a complexing ligand such as 8-hydroxyquinoline, cyclohexanediamine or N,N-dimethyl-ethylenediamine.

Optionally, the reaction is done under nitrogen environment.

Preferably, the process is performed while heating, preferably at a temperature of about 90°C to about 125°C, more preferably, of about 120°C.

The heating may be done for about 19 hours to about 72 hours, more preferably, for about 64 hours, during which the formation of 3-(trifluoromethyl)-5-(4-methyl-1H-imidazole-1-yl)-berizeneamine of formula T occurs.

Typically, the progress of the reaction can be monitored by HPLC. Preferably, the progress is determined by measuring the decrease in the amount of 3-bromo-5-trifluoromethylaniline of formula II, while heating.

Following the heating step, a cooling step is performed. Preferably, the cooling is to a temperature of about 50°C to about 40°C, more preferably, to a temperature of about 50°C to about 45°C.

The obtained 3-(trifluoromethyl-5-(4-methyl-1H-imidazole-1-yl)-benzeneamine of formula I may be recovered. The recovery can be done, for example, by quenching the reaction mixture with ammonia or ammonium hydroxide to obtain a second reaction mixture, extracting the product from the second reaction mixture; and removing the solvent from the extract.

Preferably, ammonia is added to the cooled reaction mixture at a temperature of about 45°C to about 50°C, followed by stirring for about 0.5 hour to about 1 hour, more preferably for about an hour.

Preferably, prior to extracting the product, the second reaction mixture is cooled to about room temperature.

Preferably, the extraction comprises adding water and a water-immiscible organic solvent to the second reaction mixture to obtain an organic and aqueous phase and separating the phases. Preferably, the water-immiscible organic solvent is toluene or an ester, more preferably, C₃-C₇ ester, most preferably, ethyl acetate.

The organic phase can be washed with salted water, i.e., brine, and the aqueous phase can be further extracted with a water-immiscible organic solvent.

The removal of the solvent is typically done by evaporation. The obtained residue comprising of 3-(trifluoromethyl)-5-(4-methyl-1H-imidazole-1-yl)-benzeneamine of formula I may be further crystallized.

The present invention provides crystallization processes for the purification of the compound of formula I, which avoid the need of using column chromatography.

During the process for preparing the intermediate of formula I, as described in scheme 4 the impurity 3-(trifluoromethyl)-5-(5-methyl-1H-imidazol-1-yt)benzenamine ("5 methyl isomer") of the following formula is obtained: 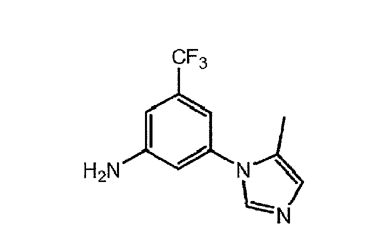 5 methyl isomer

The present invention provides a process for crystallizing the compound of formula I from a mixture of ethyl acetate and petroleumether. Typically the crystallization purifies the compound of formula I, preferably from the impurity 5 methyl isomer.

The crystallization comprises dissolving 3-(trifluorornethyl-5-(4-methyl-1H-imidazole-1-yl)-benzeneamine of formula I in ethyl acetate; adding petroleum ether to obtain a suspension; and isolating. As used herein, "petroleum ether" refers to C5-C8 hydrocarbones.

Preferably, dissolution is achieved at a temperature that is capable to dissolve formula I, more preferably, at about 45°C.

Typically, the suspension is cooled to increase the yield of the precipitated product. Preferably, the cooling is to a temperature of about 15°C to about 0°C.

The isolation may be done by filtration. The isolated compound may be further dried.

Typically, the crystallization can be repeated several times to increase the purity of the product.

Preferably, the purity of the obtained compound of formula I is about 99.7%, or greater, on area by HPLC. The purified compound of formula I contains about 0.13%, or less, on area by HPLC, of the 5 methyl isomer impurity.

The present invention further provides a process of purifying the intermediate of formula I by recrystallizing it from a mixture of isopropyl alcohol ("IPA") and water or a mixture of ethanol and water.

The preferred solvents used in this process are class 3 solvents.

Phamacopeia Forum, Vol. 29(4), pgs. 1159-1160, 2003 describes solvents of Class 2 and of Class 3. Class 2 solvents are selected from the group comprising: acetonitrile, chlorobenzene, chloroform, cyclohexane, 1,2-dichloroethene, dichloromethane, 1,2-dimethoxyethane, N,N-dimethylacetamide, N,N-dimethylformamide, 1,4-dioxane, 2-ethoxyethanol, ethylene glycol, formamide, hexane, methanol, 2-methoxyethanol, methylbutylketone, methylcyclohexane, N-methylpyrrolidone, nitromethane, pyridine, sulfolane, tetrahydrofuran, tetralin, toluene, 1,1,2-trichloroethane and xylene. Class 3 solvents are selected from the group comprising: acetic acid, acetone, anisole, 1-butanol, butyl acetate, tert-butylmethyl ether, cumene, dimethyl sulfoxide, ethanol, ethyl acetate, ethyl ether, ethyl formate, formic acid, heptane, isobutyl acetate, isopropyl acetate, methyl acetate, 3-methyl-1butanol, methylethylketone, 2-methyl-1-propanol, pentane, 1-pentanol, 1-propanol, 2-propanol and propyl acetate.

The solvents in the previously reported processes comprise, among other, solvents from Class 2. The allowed amount of Class 2 residual solvents in drug substances, excipients, and drug products is lower than the amount of solvents from Class 3, used in this invention, due to the higher toxicity of the solvents of Class 2. Therefore, the allowed amount of residual solvents in prior art processes is lower than the amount of residual solvents allowed with the solvents of this invention.

Typically, recrystallization process of the present invention is done by dissolving the compound of formula I in IPA or ethanol; heating; adding water; and cooling. Preferably, the solvent is IPA. Preferably, when the solvent used is IPA, heating is to a temperature of about 40°C to about 50°C, more preferably, to about 45°C. Preferably, when the solvent used is ethanol, heating is to a temperature of about 70°C to about 80°C, more preferably, to about reflux temperature.

The heating step is typically done while stirring.

Preferably, the water is added drop-wise.

Prior and after the cooling step, a stirring step is preferably performed.

Preferably, when the solvent used is IPA, the cooling step is done to a temperature of about 10°C to about 0°C. Preferably, when the solvent used is ethanol, the cooling step is done to about room temperature.

The obtained product may be further isolated. Preferably, the isolation is done by filtration. The obtained product may be further washed, preferably with the same solvent used in its recrystallization process.

Preferably, the purified compound of formula I contains about 2% to about 16% on area by HPLC %, of the 5 methyl isomer impurity. In the process which uses IPA, the purity is typically up to about 98% area by HPLC. In the process which uses ethanol the purity is typically up to about 86.5% area by HPLC.

The obtained 3-(trifluoromethyl)-5-(4-methyl-1H-imidazole-1-yl)-benzeneamine of formula I, obtained in the above processes, can then be used to prepare Nilotinib or a salt thereof of the following formula 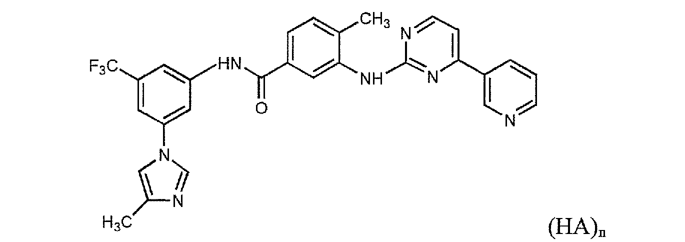 wherein, n is either 0 or 1, and HA is an acid, preferably, HCI. Typically, the conversion can be done, for example by the process disclosed in US patent no. 7,169,791, which is incorporated herein by reference. The obtained Nilotinib or salt thereof preferable contains about 0.13% or less by area on HPLC, of the 5 methyl isomer impurity.

The present invention provides N-(3-Bromo-5-trifluoromethylphenyl)-4-methyl-3-[[4-(3-pyridinyl)-2-pyrimidinyl]amino]benzamide, a new intermediate of Nilotinib of formula IV having the following structure:

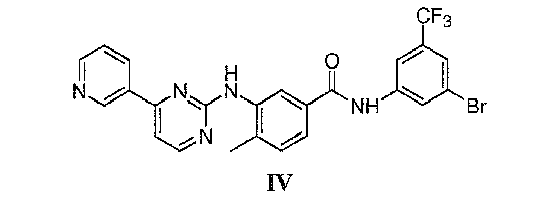

The present invention further provides isolated N-(3-Bromo-5-trifluoromethylphenyl)-4-methyl-3-[[4-(3-pyridinyl)-2-pyrimidinyl]amino]benzamide, a new intermediate of Nilotinib of formula IV.

The present invention provides a process for preparing the Nilotinib intermediate of formula IV comprising: combining a compound of formula X having the following structure: 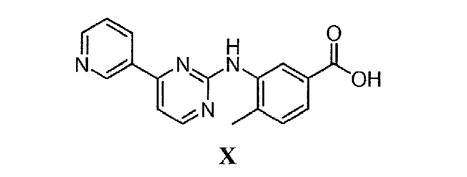 with a solvent selected from the group consisting of: aromatic hydrocarbon, a C₄-C₆ cyclic or aliphatic ether, a chlorinated solvent selected from the group consisting of: dichloromethane, dichloroethane, chlorobenzene and chloroform, DMA, DMF, DMSO and n-methyl-pyrrolidone, a compound selected from the group consisting of: thionyl chloride ("SOC1₂"), thionyl bromide, oxalyl chloride, oxalyl bromide, phosphorous trichloride, phosphorous tribromide, phosphorous pentachloride, phosphorous pentabromide, C₁-C₅ carboxylic acid and C₂-C₈ anhydride and activated reagent such as Di-tert-Butyl dicarbonate ("BOC") anhydride; a compound of formula II, and a base selected from the group consisting of: organic C₂-C₅ secondary amine such as diethylamine and dimethylamine, tertiary amines such as triethyl amine, tributylamine, diisopropyl ethyl amine and N,N-diisopropylethylamine ("DIPEA"), pyridine, 4-dimethylaminopyridine ("DMAP"), DBU, inorganic bases and mixtures thereof.

The process can be illustrated, for example, by the following scheme:

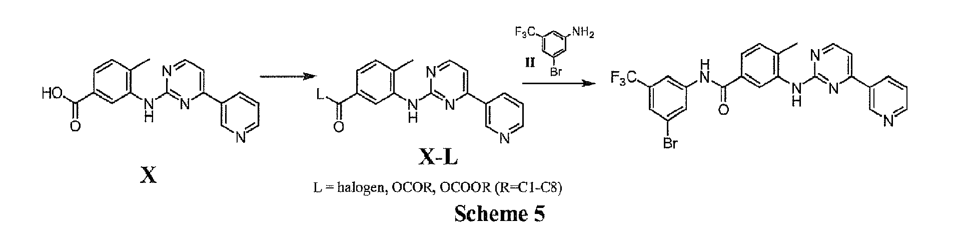

Preferably, the aromatic hydrocarbon is toluene.

Preferably, the C₄-C₆ cyclic or aliphatic ether is selected from the group consisting of: tetrahydrofuran ("THF"), methyl tert-butyl ether ("MTBE") and methyl-THF, more preferably, the C₄-C₆ cyclic ether is THF.

Preferably, the chlorinated solvent is dichloromethane.

Preferably, the compound selected from the group consisting of: thionyl chloride ("SOCl₂"), thionyl bromide, oxalyl chloride, oxalyl bromide, phosphorous trichloride, phosphorous tribromide, phosphorous pentachloride, phosphorous pentabromide C₁-C₅ carboxylic acid and C₂-C₈ anhydride and activated reagent such as Di-tert-Butyl dicarbonate ("BOC") anhydride, is SOC1₂.

Preferably, the inorganic base is selected from a group consisting of: alkali metal or alkali earth metal carbonate and bicarbonate. Preferably, the carbonate is selected from a group consisting of: K₂CO₃, Na₂COₛ, Cs₂CO₃. Preferably, the bicarbonate is selected from a group consisting of: KHCO₃, NaHCO₃.

More preferably, the base is selected from the group consisting of: N,N-diisopropylethylamine ("DIPEA"), triethylamine, tributylamine, diethylamine, 4-dimethylaminopyridine ("DMAP"), K₂CO₃, Na₂CO₃, Cs₂CO₃, NaHCO₃, KHCO₃ and combination thereof. Most preferably, the base is a mixture of DIPEA and DMAP or K₂CO₃ and DMAP.

Preferably, the process for preparing the compound of formula IV comprises: combining a compound of formula X with a solvent selected from the group consisting of: aromatic hydrocarbon, C₄-C₆ cyclic or aliphatic ether, chlorinated solvent selected from the group consisting of: dichloromethane, dichloroethane, chlorobenzene and chloroform, DMA, DMF, DMSO and n-methyl-pyrrolidone, and a compound selected from the group consisting of: SOC1₂, thionyl chloride, thionyl bromide, oxalyl chloride, oxalyl bromide, phosphorous trichloride, phosphorous tribromide, phosphorous pentachloride, phosphorous pentabromide, C₁-C₅ carboxylic acid, C₂-C₈ anhydride and activated reagent such as Di-tert-Butyl dicarbonate to obtain a first reaction mixture; adding the first reaction mixture to a second reaction mixture comprising: a compound of formula II; a base selected from the group consisting of: organic C₂-C₅ secondary amine such as diethylamine and dimethylamine, tertiary amines such as triethyl amine, tributylamine, diisopropyl ethyl amine and N,N-diisopropylethylamine ("DIPEA"), pyridine, 4-dimethylaminopyridine ("DMAP"), DBU, inorganic bases and mixtures thereof; and a solvent selected from the group consisting of: aromatic hydrocarbon, C₄-C₆ cyclic or aliphatic ether, chlorinated solvent selected from the group consisting of: dichloromethane, dichloroethane, chlorobenzene and chloroform, DMA, DMF, DMSO and n-methyl-pyrrolidone; and precipitating the compound of formula IV from a third reaction mixture.

Preferably, the solvent of the first reaction mixture is toluene.

Preferably, the solvent of the second reaction mixture is toluene or THF.

Preferably, the combining step is done under nitrogen environment.

Preferably, prior to addition of the first reaction mixture to the second reaction mixture, a heating step is performed. Preferably, the heating is to a temperature of about 45°C to about 65°C, more preferably, to about 50°C.

The first reaction mixture may be maintained prior to its addition to the second reaction mixture. Preferably, the maintaining is for about 2 hours to about overnight, more preferably, for about 12 hours.

Preferably, prior to the addition of the first reaction mixture to the second reaction mixture, the first reaction mixture is concentrated. Preferably, the concentration is to dryness to obtain a solid compound of formula X-L, wherein L is halogen, OCOR or OCOR and R is C₁-C₈ alkyl, having the following structure:

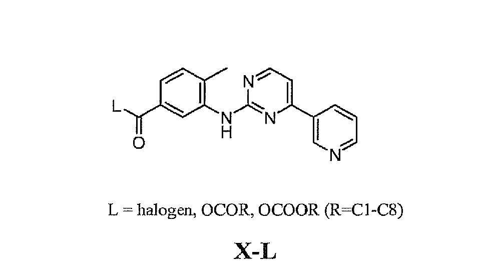

Optionally, a second portion of the same solvent used for the preparation of the first reaction mixture is added to the first reaction mixture and then removed. Preferably, the removal of the solvent is by evaporation.

The third reaction mixture, containing the compound of formula IV, is obtained from the combination of the first reaction mixture and the second reaction mixture.

Optionally, the third reaction mixture is maintained. Optionally, the maintaining step is done while stirring. The maintaining step may be done at a temperature of about 15°C to about 55°C. Preferably, the maintaining step is done at about 30°C. Preferably, the maintaining is done for about 12 hours to about overnight.

Preferably, the process for preparing the compound of formula IV comprises combining a compound of formula X with toluene, and thionyl chloride to form a first reaction mixture; adding the first reaction mixture to a second reaction mixture comprising: a compound of formula II; a mixture of DIPEA and DMAP; and THF to form a third reaction mixture; and
precipitating the compound of formula IV from the third reaction mixture.

The obtained compound of formula IV may be recovered.

Preferably, prior to the recovery step, the third reaction mixture is maintained. Optionally, the maintaining is done while stirring. The maintaining step may be done at a temperature of about 15°C to about 55°C, preferably, the maintaining step is done at about 30°C. Preferably, the maintaining is done for about 12 hours to about overnight.

The recovery can be done, for example by quenching the third reaction mixture with a base selected from the group consisting of NaHCO₃, Na₂CO₃, K₂CO₃ and KHCO₃; filtering; washing; and drying to obtain a first precipitate. Preferably, the base is Na₂CO₃ or NaHCO₃. MTBE may be added with the base as an anti-solvent, preferably when NaHCO₃ is used. Preferably, when the quenching is with Na₂CO₃, the washing is with water. Preferably, when the quenching is with NaHCO₃, the washing is with MTBE. The drying may be done in vacuum.

Alternatively, the obtained compound of formula IV may be recovered by a process comprising: adding water and a water-immiscible organic solvent to obtain a two-phase system; separating the phases; and washing the organic phase to obtain the compound of formula IV.

Preferably, the water-immiscible organic solvent is an ester, more preferably, C₃-C₇ ester, most preferably, ethyl acetate. Optionally, prior to the phase separation, the reaction mixture is stirred. Preferably, the stirring is done at about room temperature. Preferably, the stirring is done for about 10 minutes to about 30 minutes, more preferably, for about 10 minutes.

Typically, the stirring is followed by a filtration step. The washing may be done with water. Preferably, the washing is done twice. The washed organic phase is further concentrated to dryness to obtain a first precipitate containing the compound of formula IV.

Optionally, the aqueous phase is adjusted to a pH of about 7 to about 9 to obtain a second precipitate of the compound of formula IV. Preferably, the pH is about 8. Preferably, the pH is adjusted by addition of a base selected from the group consisting of: NaHCO₃, Na₂CO₃, K₂CO₃ and KHCO₃, Cs₂CO₃. Preferably, the base is NaHCO₃.

The obtained first precipitate comprising the compound of formula IV can be further combined with the filtrate, and slurried in MTBE. Typically, the slurry is filtered and dried to obtain a precipitate of the compound of formula IV. Preferably, the drying is done under vacuum. Preferably, the drying is done at a temperature of about 50°C to about 60°C, more preferably, at about 55°C.

The present invention provides another process for preparing the Nilotinib intermediate of formula IV comprising: combining a compound of formula X with a compound of formula II, a coupling reagent selected from the group consisting of: diethylcyanophosphonate, 1-hydroxybenzotriazole/ 1-[3-(Dimethylamino)propyl]-3-ethylcarbodiimide HC1 ("HOBt/EDCI") and l,l'-carbonyldiimidazole, and an aprotic polar solvent selected from the group consisting of: DMF, DMSO, dimethylacetamide ("DMA"), N-methyl pyrrolidone ("NMP") and acetonitrile. The process can be illustrated, for example, by the following scheme:

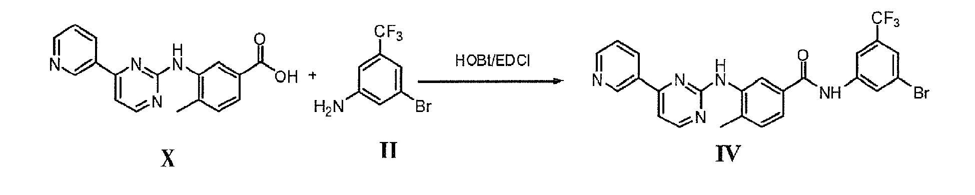

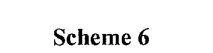

Preferably, the coupling reagent is HOBt/EDCI.

Preferably, the aprotic polar solvent is DMF.

Optionally, a base selected from the group consisting of: triethyl amine and di-isopropylethyl amine is added.

Preferably, the process is done under nitrogen environment.

Preferably, the process further comprises a heating step followed by a cooling step. Preferably, the heating is done to a temperature of about 80°C to about 100°C. Preferably, the heating step is done for about 16 hours to about 18 hours.

Preferably, the cooling is done to about room temperature.

After the cooling step, a reaction mixture containing the product of formula IV is obtained.

Preferably, the compound of formula IV is recovered. The recovery may be done by treating the reaction mixture with an aqueous solution of NaHCO3. Preferably, the reaction mixture is added to an aqueous solution of NaHCO₃.

Preferably, the purity of the obtained compound of formula IV is about 95% area by HPLC.

The present invention provides converting the compound of formula IV to Nilotinib or a salt thereof of the following formula 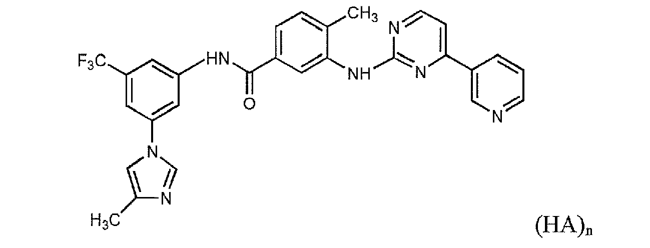 wherein, n is either 0 or 1, and HA is an acid, preferably, HCl.

The compound of formula IV may be prepared by the processes described above. The conversion may be done by combining a compound of formula IV, 4-methylimidazole of formula III, a base selected from the group consisting of: DMAP, 2,3,4,6,7,8,9,10-Octahydropyrimidol[1,2-*a*]azepine ("DBU"), K₂CO₃, Cs₂CO₃, Na₂CO₃, KHCO₃, and NaHCO₃, and a solvent selected from the group consisting of: DMSO and DMF.

Preferably, the base is DBU.

Optionally, a water absorbing agent is further added. Preferably, the water absorbing agent is selected from the group consisting of: CaO, BaO. MgO, Al₂O₃. CaO-SiC₂, CᵤSO₄, MgSO₄ and Na₂SO₄. more preferably, the water absorbent agent is CaO. Preferably, the water absorbing agent is an anhydrous salt.

Preferably, the reaction is done in the presence of an iodine salt selected from the group consisting of Cul, NaI, KI and combination thereof. Preferably, the iodine salt is Cul or NaI,

Optionally, when the iodine salt is CuI, the reaction is done in the presence of a copper complexing ligand, such as 8-hydroxyquinoline, N,N-dimethyl-ethylenediamine or mixture thereof. Preferably, the complexing ligand is a mixture of N,N-dimethylethylenediamine and 8-hydroxyquinoline.

Typically, the reaction is done under nitrogen environment.

The process may further comprise a heating step followed by a cooling step.

Preferably, heating is to a temperature of about 90°C to about 125°C, more preferably, of about 120°C. The heating step is typically done while stirring. Preferably, the heating is for a period of about 19 hours to about 72 hours, more preferably, for about 24 hours, to obtain a reaction mixture containing Nilotinib. Typically, while heating, the reaction is monitored by HPLC. The monitoring may be done by measuring the decrease in the amount of the compound of formula IV.

Preferably, the cooling is done to a temperature of about room temperature.

Preferably, the process comprises:combining: compound of formula IV, DMF, CuI, NaI, and N,N-dimethyl-ethylenediamine to obtain a reaction mixture; heating; cooling; adding 8-hydroxyquinoline, CuI, a compound of formula III and DBU; and heating,

The obtained Nilotinib may be further recovered from the reaction mixture.

The recovery may be done by treating the mixture with an aqueous solution of NaHCO₃ to obtain a second reaction mixture; extracting the product from the second reaction mixture; and removing the solvent from the extracts to obtain a residue. Preferably, the extraction comprises adding water and a water-immiscible organic solvent to the second mixture to obtain a two phase system; and separating the phases. Preferably, the water-immiscible organic solvent is an ester, more preferably, C₃-C₇ ester, most preferably, ethyl acetate.

the organic phase can be washed with salted water, i.e., brine. The aqueous phase can be further extracted with a water-immiscible organic solvent,

The removal of the solvent is typically done by evaporation. The obtained residue may be further purified.

Optionally, the process comprises combining: compound of formula IV, a solvent selected from the group consisting of: DMSO, and DMF, an iodine salt selected from the group consisting of CuI, NaI, KI and combination thereof, and N,N-dimethylethylenediamine to obtain a reaction mixture; heating; cooling; adding 8-hydroxyquinotine, an iodine salt selected from the group consisting of CuI, NaI, KI and combination thereof a compound of formula III and a base selected from the group consisting of DMAP, DBU. K₂CO₃, Cs₂CO₃, Na₂CO₃, KHCO₃. and NaHCO₃; and heating.

Preferably, heating is to a temperature of about 90°C to about 125°C, more preferably, to a temperature of about 120°C. The heating step is typically done while stirring. Preferably, the cooling is to a temperature of about 50°C to about 60°C, more preferably, to a temperature of about 60°C. The heating done after the base addition, is typically to a temperature of about 90°C to about 125°C, more preferably, of about 120°C. Preferably, the heating is for a period of about 19 hours to about 72 hours, more preferably, for about 48 hours, to obtain a reaction mixture containing Nilotinib. Typically, while heating, the reaction, is monitored by HPLC. The monitoring may be done by measuring the decrease in the amount of the compound of formula IV.

The obtained Nilotinib may be recovered from the reaction mixture. The recovery may be done by treating the reaction mixture with an aqueous solution of NaHCO₃ and water to obtain a second reaction mixture; filtering; washing and drying to obtain crude Nilotinib. Preferably, the washing is done with water. Preferably, the drying is under vacuum. The obtained crude Nilotinib may be further purified.

The present invention provides a purification process for Nilotinib comprises combining Nilotinib with DMF or methylene chloride ("DCM") to obtain a first reaction mixture; filtering; adding methanol/water to obtain a second reaction mixture; filtering; washing and drying. Preferably, prior to the methanol/water addition a washing step is performed, preferably, with DMF.

Preferably, the first reaction mixture is filtered through diatomaceous earth (e.g., Celite®). Preferably, the ratio of the methanal/water is about 3:1. Preferably, the addition of the methanol/water is done at a temperature of about 50°C to about 60°C, more preferably, at a temperature of about 60°C. Prior to the filtering of the second reaction mixture, preferably a stirring step is performed. Preferably the stirring is done at about room temperature and then at about 0°C. Preferably the stirring is done for about overnight and at about 0°C for about 2 hours. Preferably, the washing after the filtering of the second reaction mixture is done with methanol. The drying is preferably done under vacuum to obtain a precipitate.

The purification process may further comprise adding the precipitate to DMF, heating, filtering, washing with DMF, filtering, washing with MeOH and drying. The heating is done to obtain a solution. The drying is done under vacuum to obtain a pure product. Preferably, the obtained nilotinib has a purity of about 99% on area by HPLC.

Alternatively, the purification process comprises combining Nilotinib with dichloromethane/methanol to obtain a reaction mixture; filtering; and drying it.

Preferably, prior to the filtering, the reaction mixture is heated from about 30°C to about 50°C, more preferably, to about 40°C. The heating step is typically done while stirring. Preferably, the reaction mixture is heated for a period of about 0.5 hour to about 2 hours, more preferably, for about 1 hour.

Preferably, the drying step is done under vacuum. More preferably, the drying is done at a pressure of about 30mm/Hg to about 100mm/Hg.

Preferably, the obtained Nilotinib has a purity of about 98% on area by HPLC.

US patent No. 7,169,791 refers to a preparation of Nilotinib using di-ethyl cyano phosphate, which is complicated to handle, expensive and releases hazardous gases such as carbon monoxide, carbon dioxide and nitrogen oxide. The obtained yield of the Nilotinib in the above reference is low, and the processes involve multiple steps.

The present invention provides one pot process for preparing Nilotinib, which is simpler, resulting in a higher yield, without the use of di-ethyl cyano phosphate.

The present invention provides a process for preparing Nilotinib comprising: combining 3-(trifluoromethyl)-5-(4-methyl-1H-imidazole-1-yl)-benzeneamine of formula I: 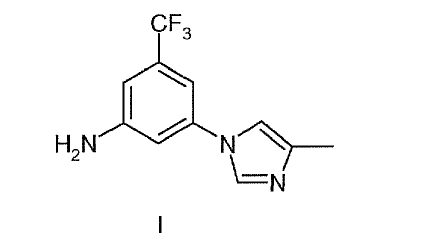 with 4-methyl-3-{[4-(pyridm-3-yl)pyrimidin-2-yl]amino}benzoic acid of formula X, having the following structure: 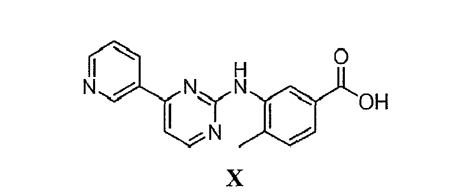 a solvent selected from the group consisting of: N-methyl pyrrolidone ("NMP"), dimethyl formamide ("DMF"), dimethylacetamide ("DMA"), and a chlorinated solvent such as CH₂C1₂, dichloroethane, and chloroform, and a compound selected from the group consisting of: thionyl chloride ("SOC1₂"), thionyl bromide, oxalyl chloride, oxalyl bromide, phosphorous tri-chloride, phosphorous tri-bromide, phosphorous penta-chloride, phosphorous penta-bromide C₁-C₅ carboxylic acid, C₂-C₈ anhydride and activated reagent such as Di-tert-Butyl dicarbonate; and adding a base selected from the group consisting of: NaOH, KOH, LiOH, K₂CO₃, Na₂CO₃, NaHCO₃, secondary amine, tertiary amine, NaH and Cs₂CO3.

Preferably, the compound is selected from the group consisting of: thionyl chloride ("SOC1₂"), thionyl bromide, oxalyl chloride, oxalyl bromide, phosphorous tri-chloride, phosphorous tri-bromide, phosphorous penta-chloride, phosphorous penta-bromide C₁-C₅ carboxylic acid, C₂-C₈ anhydride and activated reagent such as Di-tert-Butyl dicarbonate, is SOCl₂.

Preferably, the solvent is NMP.

Preferably, the base is NaOH, more preferably, an aqueous NaOH.

Typically, prior to the addition of the base, a heating step is performed. The heating may be done while stirring. Preferably, the heating is to a temperature of about 60°C to about 90°C, more preferably, to about 90°C. The heating may be done for about 2 hours to about 5 hours. More preferably, the heating is for about 3 hours.

Preferably, after the heating step and prior to the base addition, a reaction mixture is obtained and maintained at a temperature of about 25°C to about 90°C. More preferably, the reaction mixture is maintained at a temperature of about 70°C to about 90°C. Preferably, water is added prior to the maintaining step.

Optionally, prior to the maintaining step, the reaction mixture is filtered and washed. Preferably, the washing is done with a solvent selected from a group consisting of: NMP, DMF, DMA, and a chlorinated solvent such as CH₂C1₂ and water, more preferably, the washing is done with NMP and water.

The base is added until a pH of about 7.5 to about 14 is obtained. Preferably, the pH is about 10 to about 12.

After the base addition a suspension is obtained. Preferably, the suspension is further cooled. The cooling is done to a temperature of about 40°C to a temperature of about 0°C, more preferably, the cooling is to about room temperature. Typically, the cooling is done while stirring. The stirring may be done for about 15 minutes to about 2 hours, more preferably, for about 30 minutes.

Typically, after the cooling step a stirring step is performed. Preferably, the stirring is done for about 20 minutes to about 10 hours, more preferably, the stirring is done for about 2 hours. Preferably, the stirring is done at a temperature of about 35°C to about 45°C.

Optionally, the process comprises: combining the compound of formula X with a solvent selected from the group consisting of: NMP, DMF, DMA, and a chlorinated solvent such as CH₂C1₂, dichloroethane, and chloroform; heating; adding a compound selected from the group consisting of: SOCl₂, thionyl bromide, oxalyl chloride, oxalyl bromide, phosphorous tri-chloride, phosphorous tri-bromide, phosphorous penta-chloride, phosphorous penta-bromide C₁-C₅ carboxylic acid, C₂-C₈ anhydride and activated reagent such as Di-tert-Butyl dicarbonate; adding the compound of formula I; adding water; and adding a base selected from the group consisting of: NaOH, KOH, LiOH, K₂CO₃, Na₂CO₃, NaHCO₃, secondary amine, tertiary amine, NaH and Cs₂CO3. Preferably, the heating is to a temperature of about 60°C to about 90°C, more preferably, to a temperature of about 80°C. Preferably, the compound selected from the group consisting of: SOC1₂, thionyl bromide, oxalyl chloride, oxalyl bromide, phosphorous tri-chloride, phosphorous tri-bromide, phosphorous penta-chloride, phosphorous penta-bromide C₁-Cₛ carboxylic acid, C₂-C₈ anhydride and activated reagent such as Di-tert-Butyl dicarbonate, is added during about 15 minutes. Preferably, prior to the addition of the compound of formula I, a stirring step is performed. Preferably, the stirring is at a temperature of about 50°C to about 70°C, more preferably, at a temperature of about 60°C. Preferably, the stirring is done for about 1 hour to about 2 hours, more preferably, the stirring is for about 1 hour. Preferably, after the addition of water, a heating step is done. Preferably, the heating is to a temperature of about 70°C to about 90°C, more preferably, to a temperature of about 80°C.

The obtained Nilotinib may be further recovered. The recovery may be done by filtering the cooled suspension; washing; and drying. Preferably, the washing is with water. The drying may be done under vacuum. Preferably, the drying is at a temperature of about 50°C to about 60°C, more preferably, at about 50°C. The recovery may be repeated in order to increase the purity of the product.

In order to increase the yield of the obtained Nilotinib, obtained filtrate may be further treated to further recover the obtained Nilotinib. This further recovery may comprise: adding water; filtering; washing; and drying.

Preferably, prior to the filtering step, a stirring step is performed. Preferably, the stirring is done for about 0.5 hour to about 15 hours, more preferably, for about 15 hours. Preferably, the stirring is done at about room temperature. Preferably, the washing is with water. Preferably, the drying is under vacuum. Preferably, the drying is at a temperature of about 50°C to about 60°C, more preferably, at a temperature of about 50°C.

The present invention further provides a process for preparing Nilotinib HCl comprising preparing Nilotinib according to the process of the present invention and converting it to Nilotinib HCl. The conversion may be done, for example, by the process disclosed in US patent No. 7,169,791, which is incorporated herein by reference.

The present invention provides a process for preparing Nilotinib intermediate of formula (X-M)-2HM, wherein M is an halogen, having the following structure: 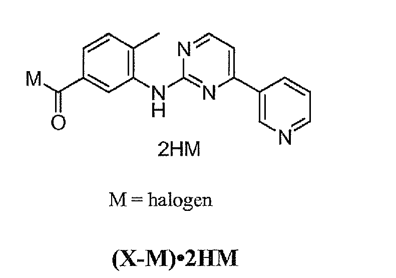 comprising: combining a compound of formula X 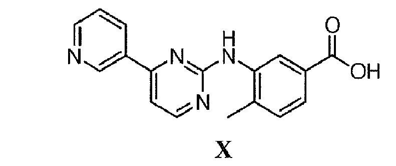 with a halogenating agent selected from the group consisting of: thionyl chloride ("SOCl₂"), phosphorous oxychloride, phosphorous penta chloride, phosphorous trichloride, sulphuryl chloride, oxalyl chloride, N-bromosuccinamide and bromine.

The process may be illustrated, for example, by the following scheme:

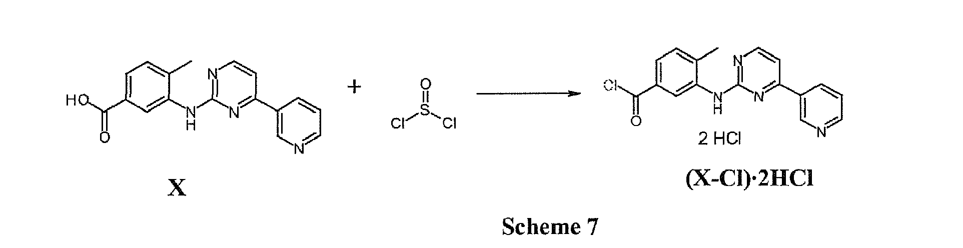

Preferably, the halogenating agent is SOC1₂.

Optionally, a solvent is added. Preferably the solvent is a non hydroxylic solvent, more preferably, the solvent is selected from the group consisting of: aromatic hydrocarbon, C₁-C₈ halogenated hydrocarbon, C₄-C₈ cyclic or aliphatic ether and mixtures thereof. Preferably the aromatic hydrocarbon is toluene or xylenes. Preferably, the halogenated hydrocarbon is selected from the group consisting of: carbon tetra chloride, chloroform, dichloromethane ("DCM"), chlorobenzene and bromobenzene. Preferably, the C₄-C₈ cyclic or aliphatic ether is selected from a group consisting of: diisopropyl ether, diethyl ether, t-butyl ether THF, MTBE, methyl-THF, dioxane and dimethoxy ethane. Most preferably, the solvent is DCM.

Optionally, the process for preparing the compound of formula (X-M)•2HM, wherein M is an halogen, is done in the presence of a catalyst. Preferably, the catalyst is a solvent in a catalytic amount. More preferably, the catalyst is DMF. As used herein, the term "catalytic amount" refers to an amount sufficient to make the reaction occur faster, preferably about 0.01 moles to about 0.2 moles, preferably to about 0.1 moles to about 0.2 moles.

Preferably, the process is done at a temperature of about 0°C to about 60°C, more preferably, at about 25°C to about 45°C, most preferably, at about 30°C to about 40°C.

Preferably, the process for preparing the Nilotinib intermediate of formula (X-M)•2HM, wherein M is an halogen, comprises: combining a compound of formula X with a halogenating agent selected from the group consisting of: SOC1₂, phosphorous oxychloride, phosphorous penta chloride, phosphorous trichloride, sulphuryl chloride, oxalyl chloride, N-bromosuccinamide and bromine; heating; and cooling.

Preferably, the heating is to a temperature of about 40°C to about reflux temperature, more preferably, the heating is to a temperature of about 60°C to about 70°C.

Preferably, prior to the cooling, a stirring step is performed. Preferably, the stirring is done for about 5 hours to about 15 hours, more preferably, for about 10 hours to about 12 hours.

Preferably, the cooling is to a temperature of about 35°C to about 15°C, more preferably, the cooling is to a temperature of about 30°C to about 27°C.

The process may further comprise a recovery step.

Optionally, prior to the recovery step, the non polar solvent, described above, is added. Preferably, the non-polar solvent is DCM. The addition of the non polar solvent is followed by a stirring step. Preferably, the stirring is performed for about 5 minutes to about 1 hour, more preferably, the stirring is performed for about 10 minutes to about 15 minutes.

Preferably, the recovery step comprises: filtering; washing; and drying.

Preferably, the washing is done with a non polar solvent, more preferably, with a solvent is selected from the group consisting of: toluene, xylene, carbon tetra chloride, chloroform, dichloromethane, chlorobenzene, bromobenzene, ether such as diisopropyl ether, diethyl ether, t-butyl ether, and mixtures thereof, most preferably, with dichloromethane. Preferably, the drying is done at a temperature of about 55°C to about 60°C. Preferably, the drying is done under reduced pressure.

The present invention provides a process for preparing Nilotinib or a salt thereof of the following formula 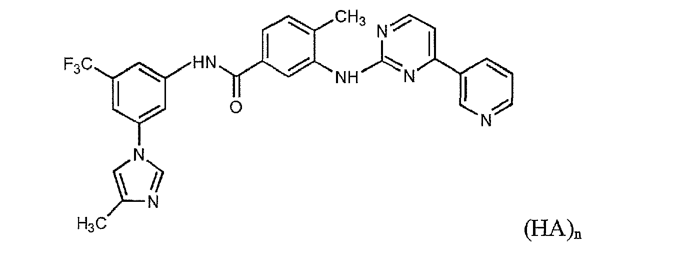 wherein n is either 0 or 1, and HA is an acid, preferably, HCl by a process comprising: preparing the compound of formula (X-M)•2HM, wherein M is an halogen; and converting it to nilotinib.

The present invention further provides 3-(4-(pyridin-3-yl)pyrimidin-2-ylamino)-N-(3-(trifluoromethyl)-5-(4-methyl-1H-imidazol-1-yl)phenyl)-4-methyl benzamide tri hydrochloride, Nilotinib•3HCl, having the following structure:

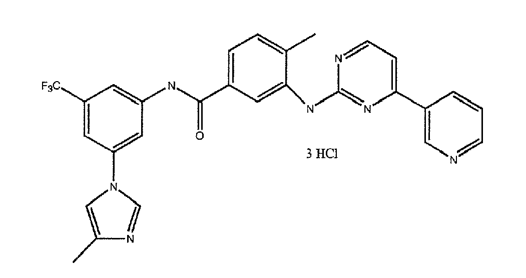

The invention encompasses a solid form of Nilotinib•3HCl.

The invention encompasses a crystalline form of Nilotinib•3HCl.

The invention encompasses a crystalline form of Nilotinib•3HCl characterized by data selected from the group consisting of: an x-ray powder diffraction pattern having peaks at about 8.3 and 25.2 degrees two theta ± 0.2 degrees two, an x-ray powder diffraction pattern substantially as depicted in Figure 3, and a combination thereof. As used herein the term "substantially" in the context of a figure relates to one skilled in the art of interpreting PXRD would consider the two diffraction patterns to represent a single isomorphic form.

The invention encompasses a crystalline form of Nilotinib•3HC1 characterized by data selected from the group consisting of an x-ray powder diffraction pattern having peaks at about 4.1,8.3 and 25.7 degrees two theta ± 0.2 degrees two theta, an x-ray powder diffraction pattern substantially as depicted in Figure 4, and a combination thereof. Typically, the crystalline form contains about 4-17% by weight water content, preferably, about 5.4-14.4% by weight water content.

The invention encompasses a crystalline form of Nilotinib•3HCl characterized by data selected from the group consisting of: an x-ray powder diffraction pattern having peaks at about 4.1, 8.3 and 25.7 degrees two theta ± 0.2 degrees two theta, an x-ray powder diffraction pattern substantially as depicted in Figure 5, and a combination thereof.

US '791 refers to a preparation of Nilotinib by condensation of the compound of formula X with the compound of formula I in the presence of diethyl cyano phosphate, which is complicated to handle, expensive and releases hazardous gases. WO '641 refers to a preparation ofNilotinib by condensation of 3-(4-(pyridine-3-yl)pyrimidin-2-ylamino)-4-methylbenzoate ester with the compound of formula I. Both condensation processes result in low yield and in the formation of impurities. The present invention provides a process for preparing Nilotinib•3HCl by condensation of 3-(4-(pyridine-3-yl)pyrimidin-2-ylamino)-4-methylbenzoyl halide of formula (X-M)2HM, wherein M is an halide, with the compound of formula I, in the absence of diethyl cyano phosphate, where the yield is high.

The invention encompasses a process for preparing Nilotinib•3HCl comprising: combining the compound of formula (X-M)•2HM, wherein M is an halogen, with the compound of formula I; and a solvent selected from the group consisting of: toluene, chloroform, dichloromethane, dimethylacetamide, THF, DMF, formamide, ethyl acetate, propyl acetate, butyl acetate, diethyl ether, methyl tert-butyl ether, hexane, cyclohexane, pentene, cyclopentene and mixtures thereof.

The process can be illustrated, for example, by the following scheme:

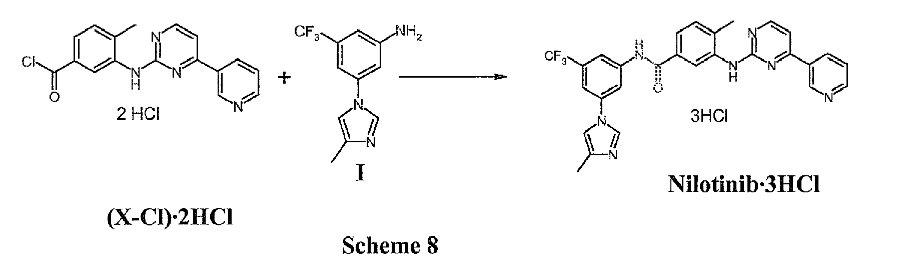

Preferably, the process is done at a temperature of about 0°C to 60°C, more preferably, at a temperature of about 25°C to about 45°C, most preferably, at a temperature of about 30°C to about 40°C.

Preferably, the process for preparing the Nilotinib•3HCl comprises: combining a compound of formula (X-M)•2HM, wherein M is an halogen, with the compound of formula I and a solvent selected from the group consisting of: toluene, chloroform, dichloromethane, dimethylacetamide, THF, DMF, formamide, ethyl acetate, propyl acetate, butyl acetate, diethyl ether, methyl tert-butyl ether, hexane, cyclohexane, pentene, cyclopentene and mixtures thereof; heating; and cooling.

Preferably, the solvent is dichloromethane, DMF or toluene. More preferably, the solvent is dichloromethane.

Preferably, the combining step is at a temperature of about 25°C to about 42°C, more preferably, at about 38°C to about 42°C.

Preferably, the heating is to about 50°C to about reflux temperature, more preferably, to about reflux temperature.

Preferably, prior to the cooling, a stirring step is performed. Preferably, the stirring is done for about 5 hours to about 15 hours, more preferably, for about 10 hours to about 12 hours. Preferably, the stirring is done at about reflux temperature.

Preferably, the cooling is to about 35°C to about 15°C, more preferably, the cooling is to a temperature from about 30°C to about 20°C.

The process may further comprise a recovery step.

Preferably, the recovery step comprises: filtering; washing; and drying. Preferably, the washing is done with the same solvent used for the reaction, more preferably, with dichloromethane. Preferably, the drying is done until constant weight, more preferably, the drying is done at about 40°C to about 60°C. Preferably, the drying is done under reduced pressure.

Preferably, the obtained Nilotinib•3HCl is characterized by data selected from the group consisting of: an x-ray powder diffraction pattern having peaks at about 8.3 and 25.2 degrees two theta ± 0.2 degrees two, an x-ray powder diffraction pattern substantially as depicted in Figure 3, and a combination thereof.

Preferably, the obtained Nilotinib•3HCl after exposure to 100% humidity for about 96 hours, having moisture at about 4.5% to about 17%, is characterized by data selected from the group consisting of: an x-ray powder diffraction pattern having peaks at about 4.1, 8.3 and 25.7 degrees two theta ± 0.2 degrees two theta, an x-ray powder diffraction pattern substantially as depicted in Figure 4 and a combination thereof.

The present invention provides another process for preparing Nilotinib•3HCl comprising: adding HCI source selected from the group consisting of: acetyl chloride, alcoholic hydrochloric acid, aqueous hydrochloric acid and hydrogen chloride gas to a first solvent selected from a group consisting of: C₁-C₄ alcohol, C₃-C₇ ketone, C₂-C₈ ester, nitrile and mixtures thereof; adding nilotinib free base; and adding a second solvent selected from the group consisting of: C₁-C₄ alcohol, C₃-C₇ ketone, C₂-C₈ ester, nitrile and mixtures thereof.

Preferably, the C₁-C₄ alcohol is methanol. The ketone may be selected from the group consisting of: acetone and methyl ethyl ketone. Preferably, the ketone is acetone.

Preferably, the first solvent is methanol.

Preferably, the second solvent is acetone.

Preferably, the first solvent is at a temperature of about -5°C to about 10°C, more preferably, at about 0°C to about 5°C.

The additions of the hydrochloric acid source and of the Nilotinib free base are done gradually, preferably, over about 15 minutes to about 30 minutes.

Prior to the addition of the second solvent, a stirring step is performed. Preferably, the stirring is for about 1 hour to about 6 hours, more preferably, for about 2 hours.

The process may further comprise a recovery step.

The recovery may be done by filtering; washing; and drying.

Preferably, the washing is done with the same second solvent used in the process. Preferably, the drying is at a temperature of about 50°C to about 75°C, more preferably, at a temperature of about 50°C to about 65°C, most preferably, at about 55°C to about 60°C. Preferably, the drying is done until constant weight, more preferably, the drying is done for about 12 hours.

The obtained compound of Nilotinib•3HCl may be further purified.

The present invention further provides a process for purifying Nilotinib•3HCl comprising: combining Nilofinib•3HCl with water; filtering to obtain a filtrate; and crystallizing.

Prior to the filtering step a charcoalizing step may be performed. When used herein the term "charcoalizing" refers to adding charcoal to the reaction mixture, preferably at reflux temperature.

Prior to the charcoalizing a heating step is performed. Preferably, the heating is to a temperature of about 40°C to about 80°C, more preferably, to a temperature of about 60°C to about 70°C.

The crystallization may be done by adding methanol to the filtrate; heating; adding water or water miscible solvent; cooling; filtering; and drying. Preferably, the water miscible solvent is selected from the group consisting of C₁-C₄ alcohol, ketone such as acetone or methyl ethyl ketone. More preferably, the water miscible solvent is acetone. Preferably, the heating is to a temperature of about 30°C to about 65 °C, more preferably, to a temperature of about 50°C to about 60°C. Preferably, the cooling is to a temperature of about 40°C to about 10°C, more preferably, to about room temperature, most preferably, to a temperature of about 30°C to about 27°C. The cooling may be followed by a stirring step. Preferably, the stirring is done for about 1 hour to about 10 hours, more preferably, for about 2 hours. Preferably, the drying is at a temperature of about 35°C to about 70°C, more preferably, at about 50°C to about 55°C. Preferably, the drying is done until constant weight. More preferably, the drying is done for about 12 hours. Preferably, the drying is done under vacuum.

Preferably, the obtained purified Nilotinib·3HC1 is characterized by data selected from the group consisting of: an x-ray powder diffraction pattern having peaks at about 4.1, 8.3 and 25.7 degrees two theta ± 0.2 degrees two theta, an x-ray powder diffraction pattern substantially as depicted in Figure 5, and a combination thereof.

Preferably, the purity of the obtained Nilotinib•3HCl is from about 95% to about 98% area % by HPLC.

The present invention further provides a process for preparing Nilotinib or a salt thereof of the following formula: 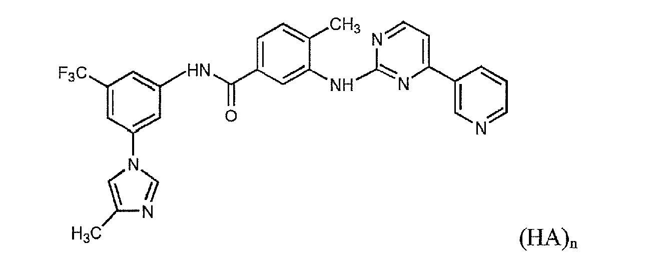 wherein, n is either 0 or 1, and HA is an acid, preferably, HCI.

The process for preparing Nilotinib or a salt thereof comprises: combining Nilotinib-3HCl with water; a base selected from the group consisting of: alkali metal hydroxide, alkali metal hydride, alkali metal carbonate, alkali metal bicarbonate, alkali metal alkoxide, C₆-C₁₀ trialkyl amines such as triethyl amine and diisopropyl ethyl amine, and pyridine; and a solvent selected from a group consisting of: methanol, ethanol, propanol, buthanol, water and mixtures thereof, to obtain a precipitate.

The alkali metal hydroxide may be NaOH, KOH and LiOH. Preferably, the base is NaOH, more preferably, the base is in methanolic solution.

Preferably the solvent is methanol.

Preferably, prior to the addition of the base, a heating step is performed. Preferably, the heating is to a temperature of about 40°C to about 80°C, more preferably, to a temperature of about 45°C to about 55°C.

Preferably, the addition of the base is done for about 1 hour to about 4 hours, more preferably, for about 1 hour to about 2 hours, to obtain a suspension.

Preferably, the suspension is heated and thereafter cooled. Preferably, the heating is to a temperature of about 40°C to about 75°C, more preferably, of about 65°C to about 70°C. Preferably, the heating is done for about 1 hour to about 8 hours, more preferably, for about 5 hours to about 6 hours. Preferably, the cooling is to a temperature of about 40°C to about 15°C, more preferably, to a temperature of about 35°C to about 30°C.

The obtained precipitate may be further recovered. The recovery may be done for example by filtering; washing; and drying. Preferably, the washing is done with water and a water miscible solvent. Preferably, the water miscible solvent is C₁-C₄ alcohol or ketone, more preferably, the water miscible solvent is methanol. Preferably, the drying is done at a temperature of about 40°C to about 60°C, more preferably, at a temperature of about 45°C to about 55°C. Preferably, the drying is done under reduced pressure.

The obtained Nilotinib can be purified.

The present invention provides a purification process of Nilotinib comprising combining it with C₁-C₄ alcohol and inorganic base selected from a group consisting of: alkali metal hydroxide, alkali metal hydride, alkali metal carbonate, alkali metal bicarbonate and alkali metal alkoxide; heating; cooling; and recovering.

Preferably, the C₁-C₄ alcohol is methanol.

Preferably, the base is NaOH.

Typically, a stirring step is performed prior to the cooling step. Preferably, the stirring is done for about 2 hours to about 8 hours, more preferably, for about 3 hours to about 4 hours.

Preferably, the cooling is to a temperature of about 40°C to about to about 20°C, more preferably, to about 40°C to about 35°C.

The recovery may be done for example by filtering; washing; and drying.

Preferably, the washing is done with C₁-C₄ alcohol. Preferably the C₁-C₄ alcohol is methanol. Preferably, the drying is done at about 45 °C to about 70°C, more preferably, at about 50°C to about 60°C. Preferably, the drying is done under vacuum. Preferably, the drying is done until constant weight. More preferably the drying is done for about 12 hours.

Preferably, the obtained Nilotinib is Nilotinib Form A as described, for example, in PCT publication no. WO 2007/015870. Nilotinib Form A is characterized by an X-ray powder diffraction pattern having at least one maxima selected from about 9.2, 13.1, 13.9, 16.7, 17.9, 18.4, 19.8, 24.1 and 25.8 degrees two theta ± 0.2 degrees two theta.

Having thus described the invention with reference to particular preferred embodiments and illustrative examples, those in the art can appreciate modifications to the invention as described and illustrated that do not depart from the spirit and scope of the invention as disclosed in the specification. The Examples are set forth to aid in understanding the invention but are not intended to, and should not be construed to limit its scope in any way.

### EXAMPLES

### HPLC method for measuring the compound of formula I_{.} the compound of formula IV and Nilotinib (according to examples 8 and 9):

### Method I:

### Reagents

Methanol (HPLC grade)
Phosphoric Acid (85%) (Analytical grade)
Water (HPLC use)
Monopotassium phosphate (Analytical grade)

### Equipment

### Agilent 1100 HPLC system (or equivalent) consisting of:

Pump: Quaternary pump
Injector: Auto sampler
Detector: Variable Wavelength Detector
Column Unit: Thermostat Column Compartment
Vacuum Degasser
Data Analysis: Agilent 1100 Chemstation

### Test Method

**Column:** Phenomen Gemini ® C18 4.6X250mm, 5µm
**Injection:** volume: 5.0µl
**Detection**: UV @ 232nm
**Column Temperature:** 40°C
**Flow rate:** 1.0ml/min
**Stop time:** 30min
**Post time**: 7min

### Mobile phase:

A: 0.02M Monopotassium phosphate solution, adjust to PH=3.5 with Phosphoric Acid
B: Methanol

### Gradient Table:

| **Time (min)** | **A** | **B** |
|---|---|---|
| 0 | 80% | 20% |
| 20 | 20% | 80 % |
| 30 | 20% | 80 % |

### Preparation of solutions

### Diluent: Methanol

Test solution: Accurately weigh 25mg of sample into a 25ml volumetric flask. Dissolve and dilute to volume with methanol, and mix well.

### Procedure

Inject diluent twice.
Inject test solution twice.

RT/ RRT of the impurity of formula I = 11.8min/ 0.83.

### Method II:

### 1. Reagents and materials

Acetonitrile (HPLC grade)
Methanol (HPLC grade)
Water (for HPLC use)
Potassium dihydrogen phosphate (Analytical grade)
85% Phosphoric acid (Analytical grade)

### 2. Equipment

### Agilent 1200 HPLC system (or equivalent) consisting of:

**Detector:** VWD
**Column Unit:** Thermostat Column Compartment
**Vacuum Degasser**
**Data Analysis:** Agilent 1200 Chemstation

### 3. HPLC conditions:

**Column:** Ultimate AQ-C 18 250mmX4.6mm, 5 µm or equivalent HPLC column
**Injection volume:** 10 µl
**Detection:** UV @ 254nm
**Column Temperature:** 30 °C
**Flow rate:** 1.0 ml/min
**Run time:** 30 min
**Post time:** 7min
**Mobile phase:**
   A: 0.01M potassium dihydrogen phosphate aqueous solution and adjust pH to 3.0 with phosphoric acid
   B: Acetonitrile

### Gradient Table:

| **Time (min)** | **A** | **B** |
|---|---|---|
| 0 | 80% | 20% |
| 20 | 20% | 80% |
| 30 | 20% | 80% |

### HPLC for measuring the compound of formula (X-C1.2HCI. Nilotinib-3HCI and Nilotinib (according to examples 18 and 19):

Column: YMC ODS AQ (150 x 4.6)mm, 5µ, YMC, P/N: AQ12S05-2546WT or equivalent.
**Flow:** 1.0 mL/min
**Injection Volume:** 10 µl
**Detector:** 240nm
**Column temperature:** 30°C
**Sample temperature:** 15°C
Diluent: Methanol
**X-ray powder diffraction:**

The x-ray powder diffraction was performed on a Bruker D8 Advance X-ray powder diffractometer, with LynxEye position sensitive detector. Copper Kα₁ radiation (λ=1.5418 Å) was used. The sample holder was a standard sample holder of PMMA with zero background plate.The scanning parameters were: range: 2-40 degrees two-theta; scan mode: continuous scan; step size: 0.05±0.005°; and time/step: O.1sec.

### Example 1: Preparation of 3-(trifluoromethyl)-5-(4-methyl-1H-imidazole-1-yl)-benzeneamine of formula I

2000g of 3-bromo-5- trifluoromethylaniline of formula II, 1368g of 4-methylimidazole of formula III , 181 g of 8-hydroxyquinoline, 238g of CuI, 666.6g of NaOH, 933g of CaO and 7000ml of DMSO were loaded into a lOL of 3-neck flask. The reaction mixture was protected with nitrogen and was then stirred at 120°C for 69 hours while monitoring for the consumption of 3-bromo-5- trifluoromethyaniline by HPLC. Heating was stopped when 3-bromo-5- trifluoromethyaniline / 4-methylimidazole is not more than 5%. The reaction mixture was cooled down to 45-50°C and poured into a 50L reactor. 8.4L of 14% ammonia was added dropwise and then stirred for 1hour at 45-50°C. The mixture was cooled down to room temperature.16.8L of water and 10L of ethyl acetate were added to the extract. The upper organic layer was separated and filtered through the filter aid. The lower aqueous layer was washed with 7.5L of ethyl acetate and combined with the above filtrate. The combined organic layer was washed with 5Lx3 of 5% of brine for three times. The upper organic layer was separated and dried over 1kg of anhydrous Na₂S0₄ overnight. The mixture was filtered and concentrated to obtain 2.3kg of solid. The residue was dissolved in 2L of ethyl acetate at 45°C. To the solution was then added 8L of petroleum ether dropwise at 45°C. The mixture was cooled down slowly to 0-15°C and stirred for 1hour. A large amount of precipitate was formed and filtered. The filtered cake was dissolved in 2L of ethyl acetate at 45°C. The solution was then added 8L of petroleum ether dropwise at 45°C. The mixture was cooled down slowly to 15-0°C and stirred for 1hour. A large precipitate was formed and filtered. The filter cake was dried at 45°C and 954g of 3-(trifluoromethyl)-5-(4-methyl-1H-imidazole-l-yl)-benzeneamine of formula I were obtained. (Yield: 47.5%). The obtained compound of formula I had purity of 99.7% on area by HPLC and contained 0.13% on area by HPLC, of the 5 methyl isomer impurity.

### Example 2: Recrystallization of 3-(trifluoromethyl)-5-(4-methyl-1H-imidazole-1-yl)-benzeneamine of formula I from IPA/water

A 50mL flask was charged with 1 g of the compound of Formula I crude (purity of 82.5%) and 3.5mL of IPA. The mixture was heated to 45°C under stirring until the entire solid dissolved. At 45°C, 6mL of water was added drop-wise. The mixture was stirred for 10min and cooled slowly to 0∼10°C. The mixture was stirred at 0∼10°C for 10 min and filtered to obtain the recrystallized compound of Formula I having a purity of 98%.

### Example 3: Recrystallization of 3-(trifluoromethyl)-5-(4-methyl-1H-imidazole-1-yl)-benzeneamine of formula I from Ethanol/water

A 50mL flask was charged with 2g of the compound of Formula I crude (purity of 83.1 %) and 4mL of Ethanol. The mixture was heated to reflux under stirring until the entire solid dissolved. While refluxing, 10 mL of water was added drop-wise. The mixture was cooled slowly to 25±5°C. The mixture was filtered and washed with a mixture of ethanol/water to obtain the recrystallized compound of Formula I having a purity of 86.5%.

The purification factor can be seen in the following table:

| | **The amount of the compound of Formula I** | **Solvent and Volume Ratio** | ***HPLC results (% on*area** | |
|---|---|---|---|---|
| | | | *RT = 11.8min Isomer (%)* | |
| | | | *Before* | *After* |
| ex.2 | 1 | IPA/water=7/12, 1 g/9.5ml | 15.45 | Not detected |
| ex. 3 | 2 g | Ethanol/water=2/5, 1 g/ 7ml | 13.17 | 8.83 |

### Example 4: Preparation of compound of formula IV

The compound of formula X **(31.0g,** 0.10mol) was suspended in 310ml toluene, and SOC1₂ (47.6g, 0.40mol) was added to the mixture under the protection of N₂. The formed mixture was reacted at 50°C for 2 h. The solvent was evaporated completely, and a compound of formula (X-C1) was obtained as yellow solid. The compound of formula (X-Cl) was then added to a THF solution of the compound of formula II (27.0g, 0.11mol), DIPEA (15.0g, 0.12mol) and DMAP (0.5g, 4.0mmol). The reaction mixture was reacted at 30°C for 12 h, and then quenched with 8% solution of sodium bicarbonate (620ml). The mixture was filtered, and washed with H₂O, then dried in vacuum. The solid was re-slurried with MTBE, and dried in vacuum again. 49.5g of the compound of formula IV were obtained as light yellow powder. The yield is about 93.7% by weight. The purity of the isolated product is 98% (% on area by HPLC).

### Example 5: Preparation of compound of formula IV

To a 50ml 3-neck flask was charged compound of formula **X** 3.1g and 21ml of toluene. The suspension was charged 5.1 1g dichlorosulfoxide (SOCl₂) under nitrogen protection. The reaction mixture was heated to 50°C and reacted for 2hrs. The reaction was then concentrated to dry. To another 100ml 3-neck flask was charged 2.7g of compound of formula II, 1.5g of DIPEA, 0.1g of DMAP and 30ml of THF. To the mixture was charged the above concentrated residue. The reaction mixture was stirred at 25±5°C overnight. The mixture was charged 45ml of ethyl acetate and 20ml of water. The mixture was then stirred at 25±5°C for 10min, filtered and the filtrate was phase separated. The organic layer was washed by water 10ml twice. Then the organic layer was concentrated to dry. The residue was combined with the filter cake and slurried in MTBE. The mixture was filtered and dried under vacuum at 50°C. The water layer was adjusted pH to 8 with NaHC0₃ solution. The second crop 0.5g was thus precipitated out. Total yield was 94%.

### Example 6: Preparation of compound of formula IV

To a 50ml 3-neck flask was charged compound of formula **X** 3.1 g, 20 mL of toluene and 18ml of dichlorosulfoxide (SOCl₂) under nitrogen protection. The reaction mixture was heated to 50°C and reacted overnight. The reaction was then concentrated to dry and co-evaporated with 20ml of toluene of once. To another 100ml 3-neck flask was charged 2.7g of compound of formula II, 1.5g of K₂CO₃, 0.1g of DMAP and toluene. To the mixture was charged the above concentrated residue. The reaction mixture was stirred at 50°C overnight. The mixture was charged 30ml of half saturated NaHCO₃ solution, 15ml of MTBE and stirred for 10min. Large amount of solid was precipitated out and filtered. The filter cake was washed with MTBE and fired under vacuum at 55 °C. The resulted product was of 81% of purity. There were about 9% of the compound of formula X.

### Example 7: Preparation of compound of formula IV

The compound of formula X (50 g), HOBt (26.5 g)/ EDCI (37.5 g) and DMF (500 mL) were loaded into a reactor at 25±5°C. After being reacted for 3h, the compound of formula II (39 g) was added to the reactor. The reaction mixture was stirred at 80°C for about 18 hours while monitoring for the consumption of active ester by HPLC. After being cooled to 25±5°C, the mixture was dropped to a solution of half-saturated aqueous solution of sodium hydrogen carbonate, and the product was precipitated as canary yellow solid.

The yield of this step was about 29.0% by weight. The purity of the isolated product was 95% (% on area by HPLC method described in Appendix 1).

### Example 8: Preparation of Nilotinib

The compound of formula IV (21.0g, 39.7mmol), NaI (12.0g, 79.8mmol), CuI (1.3g, 6.0mmol) and N,N-Dimethylethylenediamine (1.1g, 12.0mmol) were dissolved in DMF (105ml) under the protection of N₂. The formed solution was reacted at 120°C for 24h. The temperature of the above solution was decreased to 60°C.

8-Hydroxyquinoline (1.8g, 11.6mmol), CuI (1.3g, 6.0mmol), the compound of formula III (4.6g, 56.3mmol) and DBU (9.0g, 59.3mmol) were added to the above solution under the protection of N₂. The formed solution was reacted at 120°C for 48h. After the reaction was competed (detected by the consumption of the compound of formula IV, HPLC), the reaction solution was dropped to a mixture of saturated solution of NaHCO₃ (15ml) and water (300ml) at 25±5°C. The mixture was then filtered, and the filter cake was washed with water. 26.9g crude product was obtained as pale brown powder with 69% purity after drying in vacuum.

The crude product was added to 3.8 vol. DMF, and heated to dissolution. The solution was filtered through Celite, and the filter cake was washed with 0.5 vol. DMF. 3.5 vol. of methanol / H₂O (3:1) was added to the above solution at 60°C. The formed solution was stirred at 25±5°C overnight and at ice bath for 2h. The mixture was filtered, and the filter cake was washed with methanol (0.05 volx3). The first round re-crystallization solid was obtained after drying in vacuum. The above solid was added to 2.9 vol DMF, and heated to dissolution. Then filtered, and the filter cake was washed with 0.1vol. DMF. The resulting solution was stirred at 25±5°C for 0.5 h, and at ice bath for 2 h. The mixture was filtered, and the cake was washed with methanol (0.05vol×3). 9.1g solid was obtained with 99.1% purity after drying in vacuum. The total yield was about 43.5% by weight. The purity of the isolated product is 99.1 % (% on area by HPLC).

### Example 9: Preparation of Nilotinib

The compound of formula IV, the compound of formula III, Cs₂CO₃, CUI , 8-hydroxyquinoline and CaO were loaded into a reactor at 25±5°C under the protection of N₂. The reaction mixture was then stirred at 120°C for about 24 hours while monitoring for the consumption of the compound of formula IV by HPLC. After cooled to 25±5°C, the mixture was treated with a half-saturated aqueous solution of sodium hydrogen carbonate and extracted three times with ethyl acetate, then dried by Na₂SO₄. After concentration, the crude product was obtained as yellow solid. Then the solid was dissolved by CH₂Cl₂/MeOH (10 equ., 3:2), and the mixture was washed three times with water. After a period of time, the product would be crystallized from the organic solvent (purity: 95%, detected by HPLC). Few minutes later, the product would precipitate as yellow solid. Then the product was stirred in the solvent of CH₂Cl₂/MeOH (5 equ., 5:1) at 40°C for 1 hour. After that, the mixture would be filtered. The solid we got was dried in vacuum, and the product with 98% purity was obtained by this means.

The yield of this step was about 31.1 % by weight. The purity of the isolated product was 98% (% on area by HPLC method described in Appendix 1).

### Example 10: Preparation of Nilotinib:

To 250 mL glass reactor was added the compound 4-methyl-3-{[4-(pyridin-3-yl)pyrimidin-2-yl]amino}benzoic acid of formula X (10.0 g, 0.032 mol), a compound of formula 1 (8.7 g, 0.036 mol), SOCl₂ (7.5 mL, 0.103 mol) and N-Methyl-pyrrolidone (100 mL). The reaction mixture was stirred and heated to 90°C for 5 h. The reaction was then cooled to 50°C and an aqueous NaOH solution was added (12 g in 72 mL H₂O until pH 10-11. Then, the suspension was cooled to room-temperature, stirred for 30 minutes at this temperature, filtered under reduced pressure and washed with 30 mL H₂O to yield a beige solid. This material was dried under vacuum at 50°C and 8.2 g ofNilotinib base was obtained. To the mother-liquor was added H₂O (300 mL), and the mixture was stirred for 15 hours at room-temperature. A precipitate was formed and filtered under vacuum. The solid so-obtained was washed with H₂O (20 mL), and dried in vacuum oven at 50°C to yield additional 5.9 g ofNilotinib base. The total amount of Nilotinib base was 14.1 g in 81% yield.

### Example 11: Preparation of Nilotinib:

To 250 mL glass reactor was added the compound of formula 4-methyl-3-{[4-(pyridin-3-yl)pyrimidin-2-yl]amino}benzoic acid of formula **X** (20.0 g, 0.065 mol), a compound of formula **I** (17.3 g, 0.072 mol), SOCl₂ (15 mL, 0.206 mol) and N-Methyl-pyrrolidone (100 mL). The reaction mixture was stirred and heated to 90°C for 3 h. The reaction was filtered under reduced pressure and washed with NMP (10 mL) and H₂O (10 mL). The filtrate was then cooled to 70°C and a 47% NaOH solution (30 mL) was added and stirred for 30 minutes until pH 11-12. Then, the suspension was cooled to 5°C during 3 hours, stirred at this temperature for 10 hours room-temperature, filtered under reduced pressure and washed with 100 mL H₂O to yield a beige solid. This material was dried under vacuum at 50°C and 27.1 g of Nilotinib base was obtained with 76% yield. (97.2% assay, 99.17% purity).

### Example 12: Preparation of Nilotinib:

To 1L glass reactor was added the compound of formula 4-methyl-3-{[4-(pyridin-3-yl)pyrimidin-2-yl]amino}benzoic acid of formula X (80.0 g, 0.26 mol), and N-Methyl-pyrrolidone (400 mL). The mixture was heated to 60°C, then SOCl₂ (24 mL, 0.33 mol) was added during 15 minutes. The resulted mixture was stirred at 60°C for 1 h. A compound of formula **I** (69.2 g, 0.29 mol) was added and the reaction mixture was stirred and heated to 90°C for 3 h. Water (500 mL) was added and the solution was heated to 80°C. NaOH 47% solution (65 mL) was added until pH 11-12. Then, the suspension was cooled to 40°C and stirred at this temperature for 2 hours, filtered under reduced pressure at 40°C, and washed with 500 mL H₂O to yield a beige solid. This material was slurried in water (1 L) at 40°C for 1 h, filtered, washed with water (500 mL), and dried under vacuum at 50°C to obtain 135.25 g ofNilotinib base with 94% yield. (95.8% assay, 99.46% purity).

### Example 13: Preparation of 3-(4-(Pyridin-3-yl)pyrimidin-2-ylamino)-4-methylbenzoyl chloride, dihydrochloride of the formula (X-Cl)•2HCl:

Thionyl chloride (1400ML) was added to 3-(4-(pyridin-3-yl)pyrimidin-2-ylamino)-4-methylbenzoic acid of formula X (39 gms). This mixture was heated to 60-70°C and stirred for 10-12 hours. The reaction mixture was then cooled to 30-27°C. The obtained slurry was filtered and the solid was washed with dichloromethane. The wet product was dried at 55-60°C under reduced pressure.
Dry wt: 140gm
Yield: 95.4
Purity: above 98% by HPLC
Hydrochloride content (by Argentometry titration): 27.48%

### Example 14: Preparation of 3-(4-(pyridin-3-yl)pyrimidin-2-ylamino)-4-methylbenzoyl chloride, dihydrochloride of the formula (X-Cl-2HCl:

Thionyl chloride (1000ML) was added to 3-(4-(pyridin-3-yl)pyrimidin-2-ylamino)-4-methylbenzoic acid of formula **X** (100 gms). This mixture was heated to 60-70°C and stirred for 5-6 hours. The reaction mixture was then cooled to 30-35°C. Dichloromethane (1000ML) was then added to the recation mixture and stirred for 10-15 minutes.The obtained slurry was filtered and the solid was washed with dichloromethane. The wet product was dried at 55-60°C under reduced pressure.
Dry wt: 100-106gm
Purity: above 98% by HPLC

### Example 15: Preparation of Nilotinib-3HCl (crude):

3-(4-(pyridin-3-yl)pyrimidin-2-ylamino)-4-methylbenzoyl chloride dihydrochloride of formula (X-Cl)-2HCl (105 gms) was added to dichloromethane (1000ml) and 3-(trifluoromethyl)-5-(4-methyl-1H-imidazol-1-yl)benzenamine of formula I (71gms) at 25-40°C. The temperature was raised to reflux point and was stirred at this temperature for 10-12 Hours. The reaction mixture was then cooled to 30-20°C. The obtained slurry was filtered and the solid was washed with dichloromethane (200ml). The wet product was dried at 40-60 °C under reduced pressure.

The X-ray powder diffraction of the obtained product is shown in Figure 3. The X-ray powder diffraction of the obtained product after exposure to 100% humidity for 96% is shown in Figure 4.
Yield: 90-92%
Purity: 85-90%
Hydrochloride content (by Argentometry titration): 16.8%.

### Example 16: Preparation of Nilotinib-3HCl:

Methanol (50ml) was cooled to 0-5°C and acetyl chloride (2.29gm) was slowly added to it. To this mixture, 3-(4-(pyridin-3-yl)pyrimidin-2-ylamino)-N-(3-(trifluoromethyl)-5-(4-methyl-1H-imidazol-1-yl)phenyl)-4-methyl benzamide (Nilotinib free base) (5.00 gms) was added slowly and mixture was stirred for 2 hours. Acetone (50ml) was then added and mixture was stirred for 60 minutes. Reaction mass was filtered and washed with acetone (10ml). The obtained product was dried at 55-60°C.
Dry wt: 4.5gm
Yield: 75%
Purity: 95-98%

### Example 17: Purification of Nilotinib-3HCl (Pure):

3-(4-(pyridin-3-yl)pyrimidin-2-ylamino)-N-(3-(trifluoromethyl)-5-(4-methyl-1H-imidazol-1-yl)phenyl)-4-methylbenzamide tri hydrochloride (5gm) and water (25ml) were added and the mass was heated to 60-70°C. The mass was charcoalized (0.5gm carbon) and filtered through celite bed. Methanol (50ml) was added to the filtrate. The mixture was heated to 50-60°C and acetone (100ml) was added. It was then cooled to 30-27°C and stirred for 2hours. The obtained product was filtered and dried at 50-55°C for 12 hours under vacuum. The X-ray powder diffraction of the obtained product is shown in Figure 5.
Dry wt 3.5gm
Yield 0.7w/w
Purity: 95-98%

### Example 18: Preparation of Nilotinib:

3-(4-(pyridin-3-yl)pyrmidin-2-ylamino)-N-(3-(trifluoromethyl)-5-(4-methyl-1H-imidazol-1-yl)phenyl)-4-methylbenzamide tri hydrochloride (185gms) was dissolved in 825ml water and heated to 45-55°C. A methanolic solution of sodium hydroxide (35.9gm Sodium hydroxide dissolve in 1800 ml methanol) was added to the reaction mixture over a period of 1-2 hours. The suspension was heated to 65-70°C for 5-6 hours and the slurry was cooled to 35-30°C. The solid was filtered and washed with equal amount of water: methanol mixture 200ml. The wet product was dried at 45-55°C under reduced pressure.
Yield: 90%
Purity: 99.5%

### Example 19: Purification of Nilotinib:

3-(4-(pyridin-3-yl)pyrimidin-2-ylamino)-N-(3-(trifluoromethyl)-5-(4-methyl-1H-imidazol-1-yl)phenyl)-4-methylbenzamide (140gm) was taken into methanol (1.4lit) and sodium hydroxide (14gm). The mixture was heated to reflux and stirred for 3-4 hours. The mixture was the cooled to 40-35°C and filtered. The product was washed with methanol (2X50ml) and dried at 50-60°C for 12 hours under vacuum.
Dry wt. 120gm
Yield: 0.85w/w

### Example 20: Humidifying Nilotinib•3HCl (crude):

Weight 4.0 gms (having HPLC purity is 90%) Crude Nilotinib Tri hydrochloride in dry Petri dish and placed in to closed glass container containing (saturated Potassium chloride solution) at 20 to 25 temperature, the water content of crude Nilotinib was measured by regular interval.

| | |
|---|---|
| M/C Initial | 4.31% |
| After 24 Hrs | 16.33% |
| After 48 Hrs | 16.89% |
| After 72 Hrs | 16.40% |
| After 96 Hrs | 16.36% |
| After 120 Hrs | 16.53% |

### Example 21: 1 Humidifying Nilotinib-3HCl (Pure):

Weight 4.0 gms (having HPLC purity is 97.91%) Pure Nilotinib Tri hydrochloride in dry Petri dish and placed in to closed glass container containing (saturated Potassium chloride solution) at 20 to 25 temperature, the water content of Pure Nilotinib was measured by regular interval.

| | |
|---|---|
| M/C Initial | 4.55% |
| After 72 Hrs | 14.90% |
| After 96 Hrs | 14.41 % |

Further aspects and embodiments of the invention are set out in the following numbered paragraphs:
1. A one-pot process for preparing Nilotinib, comprising: combining 3-(trifluoromethyl)-5-(4-methyl-1H-imidazole-1-yl)-benzeneamine of formula I: 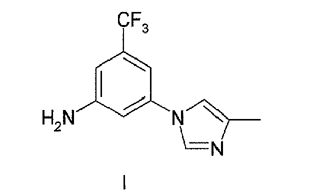 with 4-methyl-3-{[4-(pyridin-3-yl)pyrimidin-2-yl]amino}benzoic acid, of formula X, having the following structure: 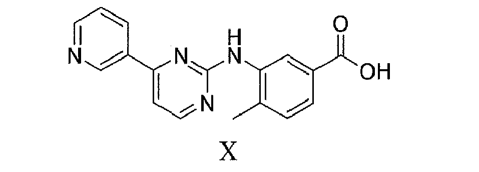 a solvent selected from the group consisting of: N-methyl pyrrolidone ("NMP"), dimethyl formamide ("DMF"), dimethylacetamide ("DMA"), and a chlorinated solvent such as CH₂Cl₂, dichloroethane, and chloroform, and a compound selected from the group consisting of: thionyl chloride, thionyl bromide, oxalyl chloride, oxalyl bromide, phosphorous tri-chloride, phosphorous tri-bromide, phosphorous penta-chloride, phosphorous penta-bromide, C₁-C₅ carboxylic acid, C₂-C₈ anhydride and Di-tert-Butyl dicarbonate; and adding a base selected from the group consisting of: NaOH, KOH, LiOH, K₂CO₃, Na₂CO₃, NaHCO₃, secondary amine, tertiary amine, NaH and C_{S2}CO₃.
2. The process of clause 1, wherein the compound is thionyl chloride.
3. The process of clause 1 or clause 2, wherein the solvent is N-methyl pyrrolidone.
4. The process of any of clauses 1-3, wherein the base is NaOH.
5. The process of any of clauses 1-4, wherein prior to the addition of the base, a heating step is performed.
6. The process of clause 5, wherein the heating is to a temperature of about 60°C to about 90°C.
7. The process of any of clauses 1-6, wherein the base is added until a pH of about 7.5 to about 14 is obtained.
8. The process of clause 7, wherein the pH is about 10 to about 12.
9. The process of any of clauses 1-8, wherein after the base addition, a suspension containing nilotinib is obtained.
10. The process of clause 9, wherein the suspension is cooled.
11. The process of clause 10, wherein the cooling is to a temperature of about 40°C to a temperature of about 0°C.
12. The process of clause 1, wherein the process comprises dissolving a compound of formula X in a solvent selected from the group consisting of: N-methyl pyrrolidone ("NMP"), dimethyl formamide ("DMF"), dimethylacetamide ("DMA"), and a chlorinated solvent such as CH₂Cl₂, dichloroethane, and chloroform; heating; adding a compound selected from the group consisting of: thionyl chloride, thionyl bromide, oxalyl chloride, oxalyl bromide, phosphorous tri-chloride, phosphorous tri-bromide, phosphorous penta-chloride, phosphorous penta-bromide, C₁-C₅ carboxylic acid, C₂-C₈ anhydride and Di-tert-Butyl dicarbonate; adding the compound of formula I; adding water; and adding a base selected from the group consisting of: NaOH, KOH, LiOH, K₂CO₃, Na₂CO₃, NaHCO₃, secondary amine, tertiary amine, NaH and Cs₂CO₃.
13. The process of clause 12, wherein after the addition of water, a heating step is done.
14. The process of clause 13, wherein the heating after the water addition is to a temperature of about 80°C to about 90°C.
15. A process for preparing Nilotinib-HCl comprising preparing Nilotinib according to the process of any of clauses 1-15, and 17 and 41-65, and 96 and converting it to Nilotinib-HCl.
16. Isolated N-(3-Bromo-5-trifluoromethylphenyl)-4-methyl-3-[[4-(3-pyridinyl)-2-pyrimidinyl]amino]benzamide, a Nilotinib intermediate of formula IV, having the following structure: 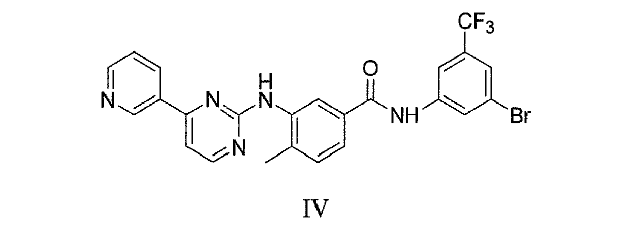
17. A process for preparing the compound of clause 16 comprising: combining a compound of formula X 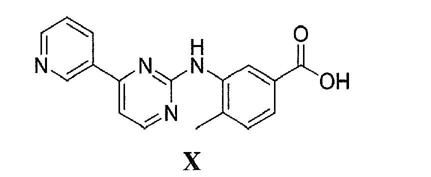 a solvent selected from the group consisting of: aromatic hydrocarbon;
   a C₄-C₆ cyclic or aliphatic ether, a chlorinated solvent selected from the group consisting of dichloromethane, dichloroethane, chlorobenzene and chloroform, DMA, DMF, DMSO and n-methyl-pyrrolidone;
   a compound selected from the group consisting of thionyl chloride, thionyl bromide, oxalyl chloride, oxalyl bromide, phosphorous trichloride, phosphorous tribromide, phosphorous pentachloride, phosphorous pentabromide, C₁-C₅ carboxylic acid ,C₂-C₈ anhydride, and Di-tert-Butyl dicarbonate;
   3-bromo-5- trifluoromethylaniline of formula II; and
   a base selected from the group consisting of organic C₂-C₅ secondary and tertiary amines, pyridine, 4-dimethylaminopyridine ("DMAP"), DBU, and inorganic bases.
18. The process of clause 17, wherein the aromatic hydrocarbon is toluene.
19. The process of clause 17, wherein the C₄-C₆ cyclic or aliphatic ether is selected from the group consisting of: tetrahydrofuran, methyl tert-butyl ether and methyl-THF.
20. The process of clause 19, wherein the ether is THF.
21. The process of clause 17, wherein the chlorinated solvent is dichloromethane.
22. The process of any of clauses 17-21, wherein the compound is SOCl₂.
23. The process of any of clauses 17-22, wherein the base is selected from the group consisting of: triethyl amine, tributylamine, diisopropyl ethyl amine and N,N-diisopropylethylamine ("DIPEA").
24. The process of clause 17, wherein the base is selected from a group consisting of:
   alkali metal or alkaline earth metal carbonate and bicarbonate.
25. The process of clause 17, wherein the base is a mixture of (a) DIPEA and DMAP or (b) K₂CO₃ and DMAP.
26. The process of any of clauses 17-25, wherein the process comprises:
   combining a compound of formula X with a solvent selected from the group consisting of: aromatic hydrocarbon, C₄-C₆ cyclic or aliphatic ether, chlorinated solvent selected from the group consisting of: dichloromethane, dichloroethane, chlorobenzene and chloroform, DMA, DMF, DMSO and n-methyl-pyrrolidone, and a compound selected from the group consisting of: thionyl chloride, thionyl bromide, oxalyl chloride, oxalyl bromide, phosphorous trichloride, phosphorous tribromide, phosphorous pentachloride, phosphorous pentabromide, C₁-C₅ carboxylic acid ,C₂-C₈ anhydride and Di-tert-Butyl dicarbonate to form a first reaction mixture;
   adding the first reaction mixture to a second reaction mixture comprising: a compound of formula II; a base selected from the group consisting of: organic C₂-C₅ secondary amine, tertiary amines, pyridine, 4-dimethylaminopyridine ("DMAP"), DBU, inorganic bases and mixtures thereof; and a solvent selected from the group consisting of: aromatic hydrocarbon, C₄-C₆ cyclic or aliphatic ether, chlorinated solvent selected from the group consisting of: dichloromethane, dichloroethane, chlorobenzene and chloroform, DMA, DMF, DMSO and n-methyl-pyrrolidone to form a third reaction mixture; and
   precipitating the compound of formula IV from the third reaction mixture.
27. The process of clause 26, wherein the solvent of the first reaction mixture is toluene.
28. The process of clause 26 or clause 27, wherein the solvent of the second reaction mixture is toluene or THF.
29. The process of any of clauses 26-28, wherein prior to addition of the first reaction mixture to the second reaction mixture, the first reaction mixture is heated to a temperature of about 45°C to about 65°C.
30. The process of any of clauses 26-29, wherein prior to the addition of the first reaction mixture to the second reaction mixture, the first reaction mixture is concentrated.
31. The process of clause 30, wherein the concentration is to dryness to obtain a solid compound of formula X-L having the following structure: 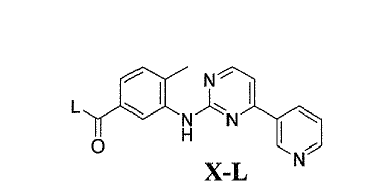 L = halogen, OCOR, OCOOR (R=Cl-C8)
   wherein L is halogen, OCOR or OCOOR and R is C₁-C₈ alkyl.
32. The process of any of clauses 26-31, wherein the process comprises:
   combining a compound of formula X with toluene, and thionyl chloride to form a first reaction mixture;
   adding the first reaction mixture to a second reaction mixture comprising: a compound of formula II; a mixture of DIPEA and DMAP; and THF to form a third reaction mixture; and
   precipitating the compound of formula IV from the third reaction mixture.
33. A process for preparing the compound of clause 16, comprising: combining a compound of formula X 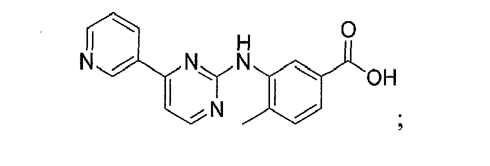 3-bromo-5- trifluoromethylaniline of formula II 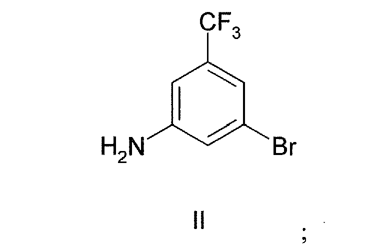 a coupling reagent selected from the group consisting of:
   diethylcyanophosphonate, 1-hydroxybenzotriazole/1-[3-(Dimethylamino)propyl-3-ethylcarbodiimide HCl ("HOBt/EDCI") and 1,1'-carbonyldiimidazole; and
   an aprotic polar solvent selected from the group consisting of: DMF, DMSO, dimethylacetamide ("DMA"), N-methyl pyrrolidone ("NMP") and acetonitrile.
34. The process of clause 33, wherein the coupling reagent is HOBt/EDCI.
35. The process of any of clauses 33-34, wherein the aprotic polar solvent is DMF.
36. The process of any of clauses 33-35, wherein a base selected from the group consisting of: triethyl amine and di-isopropylethyl amine is further added.
37. The process of any of clauses 35-36, further comprising a heating step followed by a cooling step.
38. The process of clause 37, wherein the heating is done to a temperature of about 80°C to about 100°C.
39. The process of any of clauses 37-38, wherein the cooling is done to about room temperature.
40. A process for preparing Nilotinib or salt thereof by a process comprising converting the compound of clause 16 to Nilotinib or a salt thereof.
41. The process of clause 40, wherein the conversion is done by combining a compound of formula IV, 4-methylimidazole of formula III, a base selected from the group consisting of: DMAP, DBU, K₂CO₃, C_{S2}CO₃, Na₂CO₃, KHCO₃, and NaHCO₃ and a solvent selected from the group consisting of: DMSO and DMF.
42. The process of clause 41, wherein the base is DBU.
43. The process of any of clauses 41-42, wherein a water absorbing agent is further added.
44. The process of clause 43, wherein the water absorbing agent is selected from the group consisting of: CaO, BaO, MgO, Al₂O₃, CaO-SiO₂, CuSO₄, MgSO₄ and Na₂SO₄.
45. The process of clause 43, wherein the water absorbing agent is an anhydrous salt.
46. The process of clause 44, wherein the water absorbing agent is CaO.
47. The process of any of clauses 41-46, wherein the combining step further comprises an iodine salt selected from the group consisting of CuI, NaI, KI and combination thereof.
48. The process of clause 47, wherein the iodine salt is CuI or NaI.
49. The process of any of clauses 47-48, wherein the combining step further comprises a copper complexing ligand selected from the group consisting of: 8-hydroxyquinoline, N,N'-dimethyl ethylene diamine and mixture thereof, when the iodine salt is CuI.
50. The process of clause 49, wherein the complexing ligand is a mixture of N,N'-dimethyl ethylene diamine and 8-hydroxyquinoline.
51. The process of any of clauses 41-50, further comprising a heating step followed by a cooling step.
52. The process of clause 51, wherein the heating is to a temperature of about 90°C to about 125°C.
53. The process of clause 51 or clause 52, wherein the cooling is about room temperature.
54. The process of any of clauses 41-53, wherein the process comprises combining:
   compound of formula IV, a solvent selected from the group consisting of: DMSO and DMF, an iodine salt selected from the group consisting of CuI, NaI, KI and
   combination thereof, and N,N'-dimethyl ethylene diamine to obtain a reaction mixture; heating; cooling; adding 8-hydroxyquinoline, an iodine salt selected from the group consisting of CuI, NaI, KI and combination thereof, a compound of formula III and a base selected from the group consisting of: DMAP, DBU, K₂CO₃, C_{S2}CO₃, Na₂CO₃, KHCO₃, and NaHCO₃; and heating.
55. The process of clause 54, wherein the process comprises combining: compound of formula IV, DMF, CuI, NaI, and N,N'-dimethyl ethylene diamine to obtain a reaction mixture; heating; cooling; adding 8-hydroxyquinoline, CuI, a compound of formula III and DBU; and heating.
56. A process for purifying Nilotinib comprising:
   combining Nilotinib with DMF or methylene chloride ("DCM") to obtain a first reaction mixture;
   filtering the first reaction mixture;
   combining methanol/water with the first filtered reaction mixture to obtain a second reaction mixture;
   filtering the nilotinib from the second reaction mixture;
   washing the nilotinib; and
   drying the nilotinib.
57. The process of clause 56, wherein the first reaction mixture is filtered through diatomaceous earth.
58. The process of any of clauses 56-57, wherein the addition of the methanol/water is done at a temperature of about 50°C to about 60°C.
59. The process of any of clauses 56-58, wherein the washing is done with methanol.
60. The process of any of clauses 56-59, wherein the drying is done under vacuum to obtain a solid.
61. The process of any of clauses 56-60, further comprising adding the solid to DMF, heating, filtering, washing with DMF, filtering, washing with MeOH and drying.
62. The process of clause 61, wherein the obtained Nilotinib has purity of about 99%, or greater, by area on HPLC.
63. A process for purifying Nilotinib comprising combining Nilotinib with dichloromethane/methanol to obtain a reaction mixture; filtering; and drying.
64. The process of clause 63, wherein prior to the filtering, the reaction mixture is heated from about 30°C to about 50°C.
65. The process of clause 63 or clause 64, wherein the drying step is done under vacuum.
66. A process for preparing 3-(trifluoromethyl)-5-(4-methyl-1H-imidazole-1-yl)-benzeneamine of formula I, 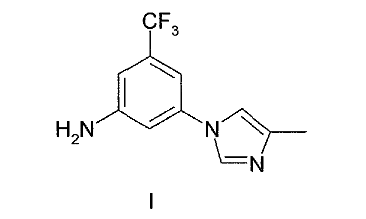 comprising combining
   (a) 3-bromo-5- trifluoromethylaniline
   (b) 4-methylimidazole
   (c) an alkali metal hydroxide; and
   (d) a water absorbing agent,
   thereby forming the compound of formula I.
67. The process of clause 66, wherein the alkali metal hydroxide is NaOH, KOH or LiOH.
68. The process of clause 67, wherein the alkali metal hydroxide is NaOH.
69. The process of any of clauses 66-68, wherein the water absorbing agent is selected from the group consisting of: CaO, BaO, MgO, Al₂O₃, CaO.SiO_{2,} CuSO_{4,} MgSO₄ and Na₂SO_{4.}
70. The process of any of clauses 66-69, wherein the water absorbing agent is an anhydrous salt.
71. The process of clause 69 wherein the water absorbing agent is CaO.
72. The process of any of clauses 66-71, wherein the process is done in the presence of a polar aprotic organic solvent.
73. The process of any of clauses 66-72, wherein the process is done in the presence of an iodine salt selected from the group consisting of CuI, NaI and KI.
74. The process of any of clauses 66-73, wherein the process is carried out in the presence of CuI and a copper complexing ligand, wherein the process is done in the presence of a copper complexing ligand when the process is carried out in the presence of CuI.
75. The process of clause 74, wherein the copper complexing ligand is 8-hydroxyquinoline.
76. The process of clause 66, comprising precipitating the compound of formula I from a reaction mixture comprising the compounds of formula II and III, a polar aprotic organic solvent, an alkaline metal hydroxide, a water absorbing agent, CuI, and a complexing ligand selected from the group consisting of 8-hydroxyquinoline, cyclohexanediamine or N,N'-dimethyl-ethylenediamine.
77. The process of clause 66, wherein the process is done at a temperature of about 90°C to about 125°C.
78. The process of clause 77, further comprising a cooling step, thereby precipitating the compound of formula I.
79. The process of clause 78, wherein the mixture is cooled to a temperature of about 50°C to about 40°C.
80. A process for purifying the compound of formula I, comprising crystallizing the compound of formula I 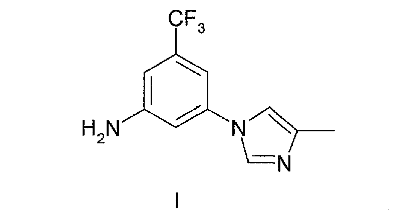 from a mixture of ethyl acetate and petroleumether.
81. The process of clause 80, wherein the process comprises dissolving 3-(trifluoromethyl-5-(4-methyl-1H-imidazole-1-yl)-benzeneamine of formula I in ethyl acetate; adding petroleumether to obtain a suspension; and optionally isolating the compound of formula 1.
82. The process of clause 80 or clause 81, wherein the purity of the obtained compound of formula I is about 99.7% or more, by area on HPLC.
83. The process of any of clauses 80-82, wherein the obtained compound of formula I contains about 0.13% or less by area on HPLC, of the 5 methyl isomer impurity.
84. The process of any of clauses 81-83, wherein the dissolving step is at a temperature at which the solvent is capable of dissolving the compound of formula 1.
85. The process of clause 84, wherein the temperature is about 45°C.
86. The process of clause 80, further comprising cooling, wherein the cooling is to a temperature of about 15°C to about 0°C.
87. A process for purifying 3-(trifluoromethyl)-5-(4-methyl-1H-imidazole-1-yl)-benzeneamine by recrystallizing it from a mixture of IPA and water or a mixture of ethanol and water.
88. The process of clause 87, wherein the recrystallization process comprises:
   dissolving the compound of formula I in IPA or ethanol; heating; adding water;
   cooling; and isolating the compound of formula 1.
89. The process of clause 88, wherein IPA is used and the heating is to a temperature of about 40°C to about 50°C.
90. The process of clause 88, wherein ethanol is used and the heating is to a temperature of about 70°C to about 80°C.
91. The process of any of clauses 88-90, wherein the water is added drop-wise.
92. The process of any of clauses 88-91, wherein IPA is used and the cooling step is done to a temperature of about 10°C to about 0°C.
93. The process of any of clauses 88-92, wherein ethanol is used and the cooling step is done to about room temperature.
94. A process for preparing Nilotinib or salt thereof of the following formula 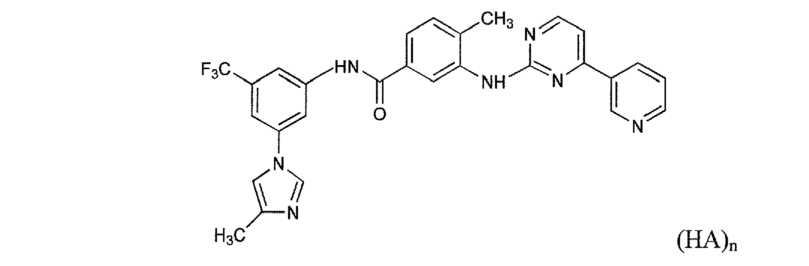 comprising preparing 3-(trifluoromethyl)-5-(4-methyl-1H-imidazole-1-yl)-benzeneamine of formula I according to the processes of any of clauses 66-93 and converting it to Nilotinib or a salt thereof, wherein, n is either 0 or 1, and HA is an acid.
95. The process of clause 94, wherein the obtained Nilotinib or salt thereof contains about 0.13% or less by area on HPLC, of the 5 methyl isomer impurity.
96. A process for preparing Nilotinib intermediate of formula (X-M)•2HM, wherein M is an halogen, comprising: combining a compound of formula X: 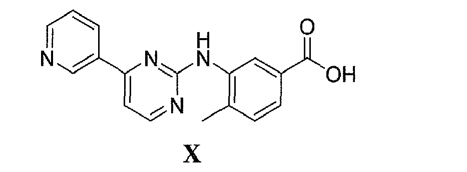 with a halogenating agent selected from the group consisting of: thionyl chloride ("SOCl₂"), phosphorous oxychloride, phosphorous penta chloride, phosphorous trichloride, sulphuryl chloride, oxalyl chloride, N-bromosuccinamide and bromine.
97. The process of clause 96, wherein the halogenating agent is SOCl₂.
98. The process of clause 96 or clause 97, wherein a solvent is present.
99. The process of clause 98, wherein the solvent is a non hydroxylic solvent.
100. The process of clause 99, wherein the non hydroxylic solvent is selected from the group consisting of: aromatic hydrocarbon, C₁-C₈ halogenated hydrocarbon, C₄-C₈ cyclic or aliphatic ether and mixtures thereof.
101. The process of clause 100, wherein the solvent is dichloromethane.
102. The process of any of clauses 96-101, wherein the process is done in the presence of a catalyst.
103. The process of clause 102, wherein the catalyst is a solvent in a catalytic amount.
104. The process of any of clauses 102-103, wherein the catalyst is DMF.
105. The process of any of clauses 96-104, wherein the process is done at a temperature of about 0°C to about 60°C.
106. The process of any of clauses 96-104, wherein the process further comprises heating and cooling steps.
107. A process for preparing Nilotinib or a salt thereof comprising: preparing the compound of formula (X-M).2HM according to the process of any of clauses 96-106 and further converting it to Nilotinib or a salt thereof.
108. 3-(4-(pyridin-3-yl)pyrimidin-2-ylamino)-N-(3-(trifluoromethyl)-5-(4-methyl-1H-imidazol-1-yl)phenyl)-4-methyl benzamide tri hydrochloride ("Nilotinib•3HCl").
109. Nilotinib•3HCl of clause 108 in solid form.
110. Nilotinib•3HCl of clause 108 or clause 109 in crystalline form.
111. The crystalline form of clause 110, characterized by data selected from the group consisting of: an x-ray powder diffraction pattern having peaks at about 8.3 and 25.2 degrees two theta ± 0.2 degrees two theta, an x-ray powder diffraction pattern substantially as depicted in Figure 3, and a combination thereof.
112. The crystalline form of clause 111, characterized by an x-ray powder diffraction pattern having peaks at about 8.3 and 25.2 degrees two theta ± 0.2 degrees two theta.
113. The crystalline form of clause 111, characterized an x-ray powder diffraction pattern substantially as depicted in Figure 3.
114. The crystalline form of clause 110, characterized by data selected from the group consisting of: an x-ray powder diffraction pattern having peaks at about 4.1, 8.3 and 25.7 degrees two theta ± 0.2 degrees two theta, an x-ray powder diffraction pattern substantially as depicted in Figure 4, and a combination thereof.
115. The crystalline form of clause 114, characterized by an x-ray powder diffraction pattern having peaks at about 4.1, 8.3 and 25.7 degrees two theta ± 0.2 degrees two theta.
116. The crystalline form of clause 114, characterized by an x-ray powder diffraction pattern substantially as depicted in Figure 4.
117. The crystalline form of clause 110, characterized by data selected from the group consisting of: an x-ray powder diffraction pattern having peaks at about 4.1, 8.3 and 25.7 degrees two theta ± 0.2 degrees two theta, an x-ray powder diffraction pattern substantially as depicted in Figure 5, and a combination thereof.
118. The crystalline form of clause 117, characterized by an x-ray powder diffraction pattern having peaks at about 4.1, 8.3 and 25.7 degrees two theta ± 0.2 degrees two theta.
119. The crystalline form of clause 117, characterized by an x-ray powder diffraction pattern substantially as depicted in Figure 5.
120. A process for preparing Nilotinib•3HCl of clause 108 comprising: combining the compound of formula (X-M)•2HM: 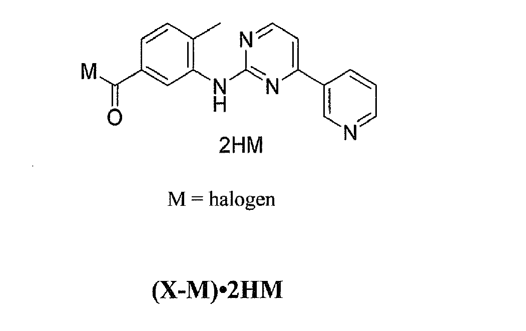 wherein M is an halogen, with the compound of formula I, and a solvent selected from the group consisting of: toluene, chloroform, dichloromethane, dimethylacetamide, THF, DMF, formamide, ethyl acetate, propyl acetate, butyl acetate, diethyl ether, methyl tert-butyl ether, hexane, cyclohexane, pentene, cyclopentene and mixtures thereof.
121. The process of clause 120, wherein the process is done at a temperature of about 0°C to about 60°C.
122. The process of clause 120 or clause 121, wherein the process further comprises a heating and a cooling step.
123. The process of any of clauses 120-122, wherein the solvent is dichloromethane, DMF or toluene.
124. The process of clause 123, wherein the solvent is dichloromethane.
125. A process for preparing Nilotinib•3HCl of clause 108 comprising: adding HCl source selected from the group consisting of: acetyl chloride, alcoholic hydrochloric acid, aqueous hydrochloric acid and hydrogen chloride gas to a first solvent selected from the group consisting of: C₁-C₄ alcohol, C₃-C₇ ketone, C₂-C₈ ester, nitrile and mixtures thereof; adding nilotinib free base; and adding a second solvent selected from the group consisting of: C₁-C₄ alcohol, ketone, ester, nitrile and mixtures thereof.
126. The process of clause 125, wherein the first solvent is methanol.
127. The process of any of clauses 125-126, wherein the second solvent is acetone.
128. The process of any of clauses 125-127, wherein the first solvent is at a temperature of about -5°C to about 10°C.
129. The process of any of clauses 125-128, wherein the hydrochloric acid source is added gradually.
130. The process of clause 125, wherein the Nilotinib free base is added gradually.
131. A process for purifying the Nilotinib•3HCl of clause 108 comprising:
   combining Nilotinib•3HCl with water; filtering to obtain a filtrate; and crystallizing.
132. The process of clause 131, wherein a charcoalizing step is done prior to the filtering step.
133. The process of clause 132, wherein prior to the charcoalizing step a heating step is performed.
134. The process of clause 133 wherein the heating is to a temperature of about 40°C to about 80°C.
135. The process of any of clauses 131-134, wherein the crystallization is done by adding methanol to the filtrate; heating; adding water or water miscible solvent; cooling; filtering; and drying.
136. The process of any of clauses 131-135, wherein the purified Nilotinib•3HCl has a purity of about 95% to about 98%.
137. Nilotinib•3HCl of any of clauses 131-136, characterized by data selected from the group consisting of: an x-ray powder diffraction pattern having peaks at about 4.1, 8.3 and 25.7 degrees two theta ± 0.2 degrees two theta, an x-ray powder diffraction pattern substantially as depicted in Figure 5, and a combination thereof.
138. A process for preparing Nilotinib or a salt thereof comprising: combining Nilotinib•3HCl of clause 108 with water; and adding a base selected from the group consisting of: alkali metal hydroxide, alkali metal hydride, alkali metal carbonate, alkali metal bicarbonate, alkali metal alkoxide, C₆-C₁₀ trialkyl amines such as triethyl amine and diisopropyl ethyl amine and pyridine; and a solvent selected from a group consisting of: methanol, ethanol, propanol, buthanol, water and mixtures thereof, to obtain a precipitate.
139. The process of clause 138, wherein the base is selected from the group consisting of NaOH, KOH and LiOH.
140. The process of clause 139, wherein the base is NaOH.
141. The process of any of clauses 138-140, wherein the solvent is methanol.
142. The process of any of clauses 138-141, wherein prior to the addition of the base a heating step is performed.
143. The process of clause 142, wherein the heating is to a temperature of about 40°C to about 80°C.
144. The process of clause any of clauses 138-143, wherein the addition of the base is done for about 1 hour to about 4 hours to obtain a suspension.
145. The process of clause 144, wherein the suspension is heated and then cooled.
146. The process of clause 145, wherein the heating is to a temperature of about 40°C to about 75°C.
147. The process of clause 145 or clause 146, wherein the cooling is to a temperature of about 40°C to about 15°C.
148. A process for purifying Nilotinib comprising: combining Nilotinib with C₁-C₄ alcohol and inorganic base selected from the group consisting of: alkali metal hydroxide, alkali metal hydride, alkali metal carbonate, alkali metal bicarbonate and alkali metal alkoxide, to form a mixture; heating the mixture; cooling the mixture; and recovering Nilotinib.
149. The process of clause 148, wherein the C₁-C₄ alcohol is methanol.
150. The process of any of clauses 148-149, wherein the base is NaOH.
151. The process of any of clauses 148-150, wherein the cooling is to a temperature of about 40°C to about to about 20°C.
152. The process of any of clauses 148-151, wherein the recovering step comprises filtering; washing; and drying.
153. The Nilotinib obtained by the process of any of clauses 148-152, wherein the Nilotinib is form A.

## Claims

1. 3-(4-(pyridin-3-yl)pyrimidin-2-ylamino)-N-(3-(trifluoromethyl)-5-(4-methyl-1H-imidazol-1-yl)phenyl)-4-methyl benzamide tri hydrochloride ("Nilotinib•3HCl").

2. Nilotinib•3HCl of claim 1 in solid form, preferably in crystalline form.

3. The crystalline form of claim 2, **characterized by** data selected from the group consisting of: an x-ray powder diffraction pattern having peaks at about 8.3 and 25.2 degrees two theta ±0.2 degrees two theta, an x-ray powder diffraction pattern substantially as depicted in Figure 3, and a combination thereof.

4. The crystalline form of claim 2, **characterized by** data selected from the group consisting of: an x-ray powder diffraction pattern having peaks at about 4.1, 8.3 and 25.7 degrees two theta ± 0.2 degrees two theta, an x-ray powder diffraction pattern substantially as depicted in Figure 4, and a combination thereof.

5. The crystalline form of claim 2, **characterized by** data selected from the group consisting of: an x-ray powder diffraction pattern having peaks at about 4.1, 8.3 and 25.7 degrees two theta ± 0.2 degrees two theta, an x-ray powder diffraction pattern substantially as depicted in Figure 5, and a combination thereof.

6. A process for preparing Nilotinib•3HCl of claim 1 comprising: combining the compound of formula (X-M)•2HM: 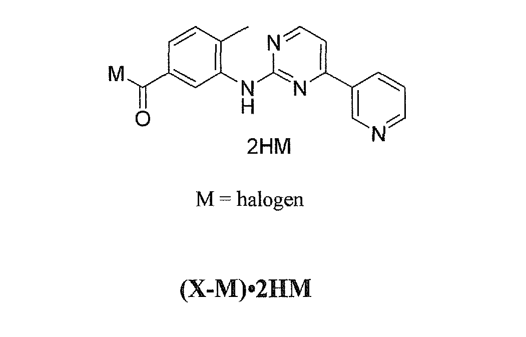 wherein M is an halogen, with the compound of formula I, and a solvent selected from the group consisting of: toluene, chloroform, dichloromethane, dimethylacetamide, THF, DMF, formamide, ethyl acetate, propyl acetate, butyl acetate, diethyl ether, methyl tert-butyl ether, hexane, cyclohexane, pentene, cyclopentene and mixtures thereof, preferably wherein the solvent is dichloromethane, DMF or toluene, preferably wherein the solvent is dichloromethane, optionally the process further comprises a heating and a cooling step.

7. The process of claim 6, wherein the process is done at a temperature of about 0°C to about 60°C.

8. A process for preparing Nilotinib•3HCl of claim 1 comprising: adding preferably adding gradually, HCl source selected from the group consisting of: acetyl chloride, alcoholic hydrochloric acid, aqueous hydrochloric acid and hydrogen chloride gas to a first solvent selected from the group consisting of: C₁-C₄ alcohol preferably methanol, C₃-C₇ ketone, C₂-C₈ ester, nitrile and mixtures thereof; adding nilotinib free base; and adding a second solvent selected from the group consisting of: C₁-C₄ alcohol, ketone preferably acetone, ester, nitrile and mixtures thereof.

9. The process of claim 8, wherein the first solvent is at a temperature of about -5°C to about 10°C.

10. The process of claim 8, wherein the Nilotinib free base is added gradually.

11. A process for purifying the Nilotinib•3HCl of any one of claims 1 to 5, preferably to a purity of about 95% to about 98%, comprising: combining Nilotinib•3HCl with water; filtering to obtain a filtrate; and crystallizing.

12. The process of claim 11, wherein a charcoalizing step is done prior to the filtering step, preferably wherein prior to the charcoalizing step a heating step is performed, preferably the heating is to a temperature of about 40°C to about 80°C.

13. The process of any of claims 11-12, wherein the crystallization is done by adding methanol to the filtrate; heating; adding water or water miscible solvent; cooling; filtering; and drying.

14. A process for preparing Nilotinib or a salt thereof comprising: combining Nilotinib•3HCl of any one of claims 1 to 5 with water; and adding a base selected from the group consisting of: alkali metal hydroxide such as NaOH, KOH or LiOH, alkali metal hydride, alkali metal carbonate, alkali metal bicarbonate, alkali metal alkoxide, C₆-C₁₀ trialkyl amines such as triethyl amine and diisopropyl ethyl amine and pyridine; and a solvent selected from a group consisting of: methanol, ethanol, propanol, buthanol, water and mixtures thereof, to obtain a precipitate.

15. The process of claim 14, wherein prior to the addition of the base a heating step is performed, preferably wherein the heating is to a temperature of about 40°C to about 80°C.

16. The process of claim 14 or claim 15, wherein the addition of the base is done for about 1 hour to about 4 hours to obtain a suspension, preferably wherein the suspension is heated and then cooled, preferably wherein the heating is to a temperature of about 40°C to about 75°C and/or the cooling is to a temperature of about 40°C to about 15°C.
